# FASCICULE DE BREVET EUROPEEN

(11) **EP 2 922 523 B1**
(45) Date de publication et mention de la délivrance du brevet: **12.09.2018**
(21) Numéro de dépôt: 13803182.8
(22) Date de dépôt: 26.11.2013
(51) Int. Cl.: A61K 8/33, A61K 8/34, A61K 8/37, A61Q 13/00, A61K 8/92, A61K 8/06, C11B 9/00, A61K 8/36, A61K 8/41, A61K 8/44, A61K 8/46, C11D 1/72, A61K 8/39, C11D 3/20, C11D 3/43, C11D 3/50, C11D 17/00

(54) **MICROEMULSIONS AQUEUSES VOLATILES DE PARFUMS ET D'HUILES ESSENTIELLES BASEES SUR L'UTILISATION DE SOLVO-SURFACTIFS**
FLÜCHTIGE WÄSSRIGE MIKROEMULSIONEN AUS DUFTSTOFFEN UND ÄTHERISCHEN ÖLEN AUF BASIS DER VERWENDUNG VON SOLVOTENSIDEN
VOLATILE AQUEOUS MICROEMULSIONS OF PERFUMES AND ESSENTIAL OILS BASED ON THE USE OF SOLVO-SURFACTANTS

(30) Priorité: 26.11.2012 FR 1203187
(43) Date de publication de la demande: 30.09.2015
(73) Titulaire: Université de Lille 1 Sciences et Technologies, 59655 Villeneuve d'Ascq Cedex (FR)
(72) Inventeur: RATAJ NARDELLO, Véronique, F-59710 Pont-a-Marcq (FR); AUBRY, Jean-Marie, F-62590 Oignies (FR)
(74) Mandataire: Grosset-Fournier, Chantal Catherine
(86) Numéro de dépôt international: PCT/FR2013/052859
(87) Numéro de publication internationale: WO 2014/080150

(56) Documents cités:
- EP-A1- 0 572 080
- WO-A1-2009/029046
- US-A- 5 585 343
- US-A1- 2008 003 247
- US-A1- 2012 097 754
- BOUTON ET ALL: "A QSPR Model for the prediction of the "fish tail" temperature of CiE4/Water/Polar hydrocarbon oil systems", LANGMUIR, vol. 26, no. 11, 5 novembre 2010 (2010-11-05), pages 7962-7970, XP002712937,
- ZHU ET ALL: "Amphiphilic properties of hydrotropes derived from isosobide : endo/exo isomeric effets and temperature dependence", LANGMUIR, vol. 25, no. 23, 9 septembre 2009 (2009-09-09), pages 13419-13425, XP002712938,

## Description

L'invention concerne des microémulsions aqueuses volatiles de parfums et d'huiles essentielles basées sur l'utilisation de solvo-surfactifs.

L'invention concerne aussi des microémulsions aqueuses volatiles d'huiles hydrophobes basées sur l'utilisation de solvo-surfactifs.

En général, les solvants des parfums sont des alcools, tels que l'éthanol ou encore l'isopropanol. L'utilisation de ces solvants présente, cependant, un certain nombre d'inconvénients : ils sont très volatils et inflammables, entraînant alors une certaine dangerosité lors de leur production, et dans une certaine mesure, de leur utilisation. D'autre part, leur propre odeur peut interférer avec celle du parfum. Enfin, appliqués sur la peau ou les cheveux, ces parfums peuvent entraîner un dessèchement, en particulier chez les consommateurs à peau sensible. Un autre inconvénient, environnemental et écologique, lié à leur appartenance à la classe des Composés Organiques Volatils (COV), est à l'origine, dans le monde entier, d'efforts pour les supprimer des compositions parfumantes. De plus, pour des raisons religieuses, certaines personnes ne consomment pas ces parfums contenant de l'alcool.

On assiste donc actuellement, pour des raisons notamment de santé publique, d'écologie, à l'émergence de nouvelles compositions. L'objectif poursuivi est la diminution voire l'élimination des COV contenus dans les parfums en développant des compositions parfumantes sous forme de solutions ou de dispersions aqueuses stables.

Cependant, la plupart des molécules parfumantes sont hydrophobes et ne sont donc pas solubles dans l'eau. Pour contourner ce problème, on doit donc utiliser des tensioactifs permettant de solubiliser les molécules parfumantes à l'intérieur de micelles ou de nanodomaines rencontrés dans des microémulsions. Il est souhaitable que les microdomaines contenant les parfums soient de petite taille pour que la composition parfumante ait un aspect transparent ou tout au moins translucide. On s'attachera donc à produire des microémulsions répondant à ce critère d'aspect transparent, plutôt que des macroémulsions. D'autres contraintes sont liées à la stabilité thermodynamique de la microémulsion, au caractère non-collant de celle-ci et à l'absence de résidu sur la peau ou sur les vêtements. Il est donc important de pouvoir les préparer en utilisant le moins de tensioactif possible.

Il est difficile de s'affranchir de tout COV. Miller et al (US2004/0068050) utilisent des diols, notamment le 1,2-hexanediol, afin d'utiliser peu de tensioactif. Mais ce diol est classé COV aux Etats-Unis. De plus, Miller *et al* indique que la suppression de polyéthylène glycol (PEG) de la composition et son remplacement par de l'eau ne conduit pas à une émulsion. Il n'est donc pas facile de préparer une microémulsion sans PEG.

Komesvarakul et al (WO2006/074177) utilisent nécessairement un composé possédant des propriétés tensioactives, tels que les esters de sorbitan (SPAN), les esters de sorbitan polyhydroxylés (TWEEN) ou certains alkylglycosides ou alkylsulfonates, pour obtenir des microémulsions aqueuses. Il n'est donc pas évident d'obtenir des émulsions aqueuses, transparentes, sans ce type de composés.

Kim et al (WO2009/029046) décrivent des microémulsions obtenues en utilisant des tensioactifs à base de sucres en présence d'un polyol. Il n'est donc pas évident d'obtenir des microémulsions aqueuses sans l'association de ces composés.

Behan et al (US1994/5374614) décrivent des compositions parfumantes aqueuses préparées en utilisant 0,5 à 50% de co-surfactant, en particulier d'alcools gras polyéthoxylés dont le déhydol 04, mais le pourcentage de parfum n'excède pas 5%, sinon ces systèmes ne sont pas transparents, alors que la transparence est hautement souhaitée dans l'industrie du parfum.

Guénin et al (US1995/5468725) décrivent des microémulsions aqueuses dans lesquelles le pourcentage massique de composition parfumante n'excède pas 3%.

Vlad et al (WO2006/043177 et US2010/7846889) décrivent des compositions transparentes à condition d'utiliser un ingrédient de solubilisation, tel que par exemple l'ajidew NL-50 (acide pyrrolidone carboxylique). Il n'est donc pas évident de pouvoir se passer d'un agent de solubilisation.

Behan et al (EP-A-0 572 080) décrivent des microémulsions à base de parfum, de tensioactifs et de solvo-surfactifs, dont des alcools gras aliphatiques en C6-C20 polyéthoxylés (2-30 EO).

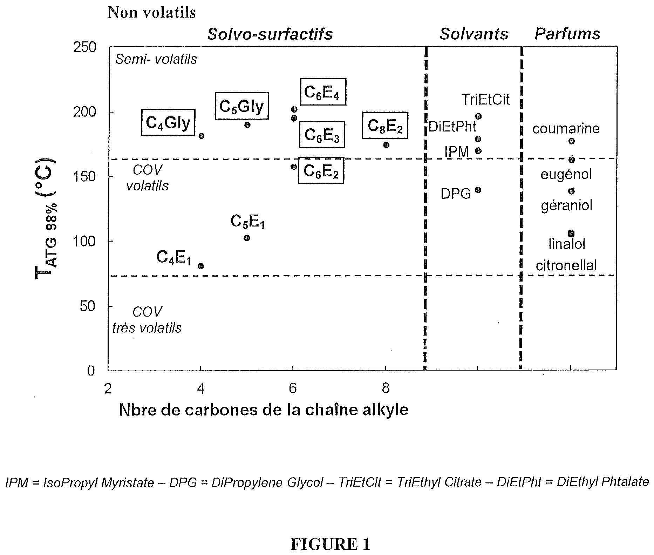

L'invention a pour but de fournir des microémulsions aqueuses, transparentes, contenant au moins 3% de parfum, et préférentiellement environ 10% de parfum, et contenant des substances volatiles éventuellement non-COV.
Selon l'invention, une substance est « volatile » lorsque sa température d'ébullition est inférieure à 250 °C à pression atmosphérique ; les composés « non-volatils » ont une température d'ébullition supérieure à 250 °C à pression atmosphérique (figure 1).

L'invention a pour but de fournir des microémulsions odoriférantes ou parfumantes comprenant le moins possible de substances provoquant des effets indésirables, en particulier sur la peau et/ou sur l'environnement. Le but de la présente invention est donc de fournir des microémulsions ayant des propriétés parfumantes stables, en limitant l'utilisation de substances non volatiles qui laissent des traces et de composés organiques volatils néfastes pour l'environnement ou l'utilisateur, dans leur préparation.

L'invention a aussi pour but de décrire des microémulsions aqueuses de parfums, stables, en particulier thermodynamiquement, non-collantes et ne laissant pas de résidu sur la peau ou sur les vêtements.
L'invention a notamment pour objet des microémulsions de type huile/eau consistant en, en pourcentage massique :
- au moins 80% d'eau,
- 1% à 10% de substances hydrophobes, en particulier 3% à 10%, notamment environ 10%,
- 5% à 15%, notamment 5% à 10%, d'un ou de plusieurs solvo-surfactifs dont la température d'ébullition est inférieure à 250 °C à pression atmosphérique,
- 0,1% à 2% d'un ou plusieurs tensioactifs, en pourcentage massique,
la composition contenant la substance hydrophobe, l'eau, le solvo-surfactif et le tensioactif contenant au plus 2 % par rapport à la masse totale de la composition de composés dont la température d'ébullition est supérieure à 250 °C à pression atmosphérique,
un des solvo-surfactifs étant un composé amphiphile choisi parmi :
▪ les alcools gras polyéthoxylés CᵢEⱼ à chaîne carbonée linéaire, de formule CH₃-(CH₂)ᵢ₋₁-(O-CH₂-CH₂)ⱼ-OH,
ou les alcools gras polyéthoxylés à chaîne ramifiée, de formule CᵢH₂ᵢ₊₁(OCH₂CH₂)ⱼOH, dans lesquelles
- i représente le nombre d'atomes de carbone de la chaîne hydrocarbonée linéaire ou ramifiée et varie de 4 à 6,
- j représente le nombre d'oxydes d'éthylène et varie de 2 à 4,
lequel tensioactif est choisi parmi
- les tensioactifs anioniques,
- les tensioactifs cationiques,
- les tensioactifs amphotères, et
   - les tensioactifs non-ioniques.

La présente invention a également pour objet des microémulsions de type huile/eau consistant en, en pourcentage massique :
- au moins 80% d'eau,
- 1% à 10% de substances hydrophobes, en particulier 3% à 10%, notamment environ 10%,
- 5% à 15%, notamment 5% à 10%, d'un ou de plusieurs solvo-surfactifs dont la température d'ébullition est inférieure à 250 °C à pression atmosphérique,
- 0,1% à 2% d'un ou plusieurs tensioactifs, en pourcentage massique,
la composition contenant la substance hydrophobe, l'eau, le solvo-surfactif et le tensioactif contenant au plus 2 % par rapport à la masse totale de la composition de composés dont la température d'ébullition est supérieure à 250 °C à pression atmosphérique,
un des solvo-surfactifs étant un composé amphiphile choisi parmi :
C₄E₁, C₅E_{1,} C₅E₂, C₅E₃, C₆E₂, C₆E₃, C₆E₄,
lequel tensioactif est choisi parmi
- les tensioactifs anioniques,
- les tensioactifs cationiques,
- les tensioactifs amphotères, et
- les tensioactifs non-ioniques.

En général, la présente description concerne des microémulsions de type huile/eau consistant en,
- de 80% à 94% d'eau, en pourcentage massique, par rapport à la masse totale de l'émulsion
- 1% à 15% en particulier 3% à 10%, notamment environ 10%, de substances hydrophobes, en particulier de composition parfumante,
- 5% à 15%, notamment 5% à 10%, d'un ou de plusieurs solvo-surfactifs volatils,
- et 0,1% à 2% d'un ou plusieurs tensioactifs, de préférence de 0,2% à 2% d'un ou plusieurs tensioactifs et de façon encore plus préférée de 0,5% à 2% d'un ou plusieurs tensioactifs, en pourcentage massique, et par rapport à la masse totale de la composition. lequel solvo-surfactif est un composé amphiphile choisi parmi :
   ▪ les alcools gras polyéthoxylés CᵢEⱼ à chaîne carbonée linéaire, de formule CH₃-(CH₂)ᵢ₋₁-(O-CH₂-CH₂)ⱼ-OH,
      ou les alcools gras polyéthoxylés à chaîne ramifiée, de formule CᵢH₂ᵢ₊₁(OCH₂CH₂)ⱼOH,
      dans lesquelles
      - i représente le nombre d'atomes de carbone de la chaîne hydrocarbonée linéaire ou ramifiée et varie de 3 à 8, notamment varie de 4 à 6,
      - j représente le nombre d'oxydes d'éthylène et varie de 1 à 4, notamment varie de 2 à 4,
   ▪ les dérivés de glycérol CₖGly de formule 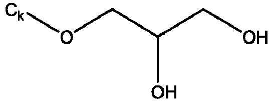 dans laquelle Cₖ représente
      - un groupe alkyle, linéaire ou ramifié, contenant de 1 à 6 atomes de carbone, en particulier de 3 à 5 atomes de carbone, notamment 4 ou 5 atomes de carbone, notamment C₄Gly de formule 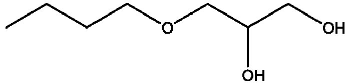 ou C₅Gly de formule 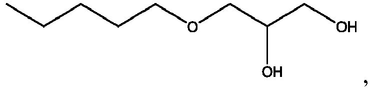 ou notamment le dérivé ramifié i-C₅Gly de formule 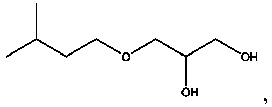
      - un groupe acyle, linéaire ou ramifié, contenant de 2 à 6 atomes de carbone, en particulier de 3 à 5 atomes de carbone, notamment 4 ou 5 atomes de carbone, notamment le pentanoate de glycéryle de formule 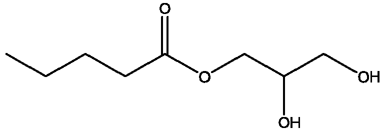
   ▪ les dérivés d'isosorbide, IsosorbideCₘ₁ et IsosorbideCₘ₂, les dérivés d'isomannide, IsomannideCₘ₃, les dérivés d'isoidide, IsoidideCₘ₄ de formules 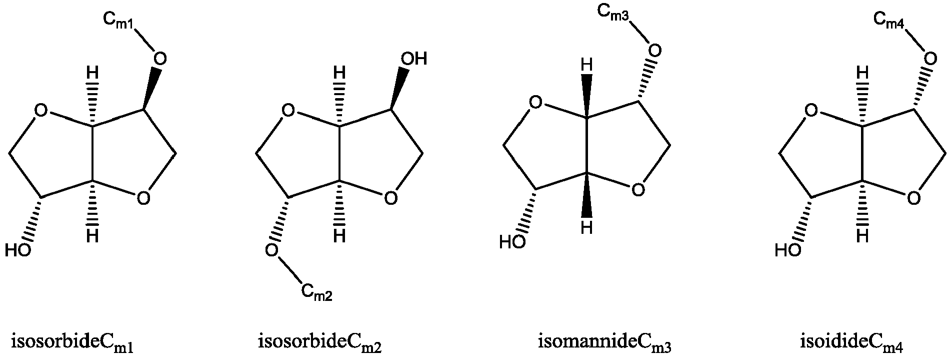 dans lesquelles Cₘ₁, Cₘ₂, Cₘ₃ et Cₘ₄ représentent
      - un groupe alkyle, linéaire ou ramifié, contenant de 1 à 6 atomes de carbone, en particulier de 3 à 5 atomes de carbone, notamment 4 ou 5 atomes de carbone,
      - un groupe acyle, linéaire ou ramifié, contenant de 2 à 6 atomes de carbone, en particulier de 3 à 5 atomes de carbone, notamment 4 ou 5 atomes de carbone,
      lequel tensioactif est choisi parmi
      - les tensioactifs anioniques,
      - les tensioactifs cationiques,
      - les tensioactifs amphotères,
      - les tensioactifs non-ioniques.

Selon un mode de réalisation avantageux, la présente description concerne des microémulsions de type huile/eau consistant en,
- 80% à 94% d'eau,
- 1% à 15% en particulier 3% à 10%, et notamment 5% à 10%, notamment environ 10%, de substances hydrophobes, en particulier de composition parfumante,
- 5% à 15%, notamment 5% à 10%, d'un ou de plusieurs solvo-surfactifs volatils,
- et 0,2% à 2% d'un ou plusieurs tensioactifs, de préférence de 0,5 à 2% d'un ou plusieurs tensioactifs en pourcentage massique,
le mélange de solvo-surfactif et de tensioactif ne contenant pas plus de 2% de composés non-volatils,
lequel solvo-surfactif est un composé amphiphile choisi parmi :
▪ les alcools gras polyéthoxylés CᵢEⱼ à chaîne carbonée linéaire, de formule CH₃-(CH₂)ᵢ₋₁-(O-CH₂-CH₂)ⱼ-OH,
   ou les alcools gras polyéthoxylés à chaîne ramifiée, de formule CᵢH₂ᵢ₊₁(OCH₂CH₂)ⱼOH,
   dans lesquelles
   - i représente le nombre d'atomes de carbone de la chaîne hydrocarbonée linéaire ou ramifiée et varie de 3 à 6, de préférence de 3 à 5 et encore plus préférentiellement est égal à 4
   - j représente le nombre d'oxydes d'éthylène et varie de 1 à 4, notamment varie de 2 à 4, et de préférence est égal à 4.
   Selon un mode de réalisation préféré, j est égal à 4 ou 2 quand i est égal à 6 ou 5 ou 4
▪ les dérivés de glycérol CₖGly de formule 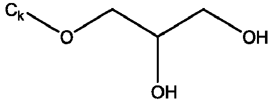 dans laquelle Cₖ représente
   - un groupe alkyle, linéaire ou ramifié, contenant de **1** à 6 atomes de carbone, en particulier de 3 à 5 atomes de carbone, notamment 4 ou 5 atomes de carbone, notamment C₄Gly de formule 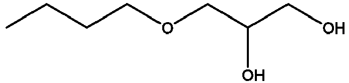 ou C₅Gly de formule 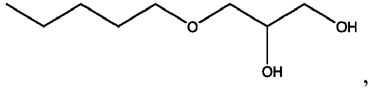 ou notamment le dérivé ramifié i-C₅Gly de formule 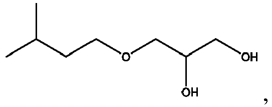
   - un groupe acyle, linéaire ou ramifié, contenant de 2 à 6 atomes de carbone, en particulier de 3 à 5 atomes de carbone, notamment 4 ou 5 atomes de carbone, notamment le pentanoate de glycéryle de formule 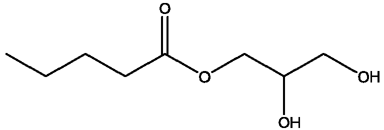
▪ les dérivés d'isosorbide, IsosorbideCₘ₁ et IsosorbideCₘ₂, les dérivés d'isomannide, IsomannideCₘ₃, les dérivés d'isoidide, IsoidideCₘ₄ de formules 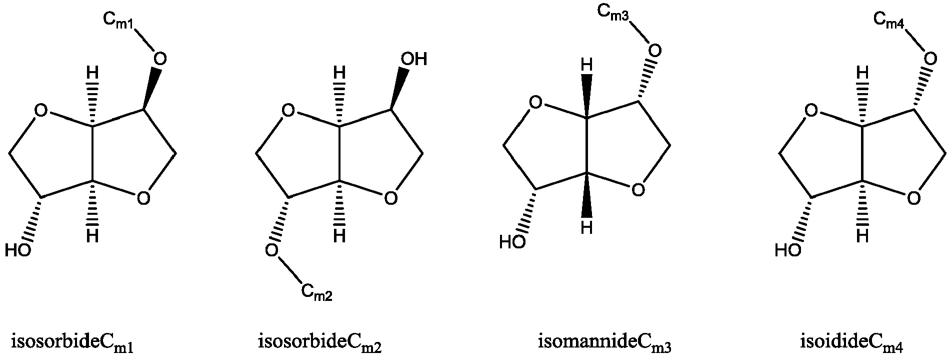 dans lesquelles Cₘ₁, Cₘ₂, Cₘ₃ et Cₘ₄ représentent
   - un groupe alkyle, linéaire ou ramifié, contenant de 1 à 6 atomes de carbone, en particulier de 3 à 5 atomes de carbone, notamment 4 ou 5 atomes de carbone,
   - un groupe acyle, linéaire ou ramifié, contenant de 2 à 6 atomes de carbone, en particulier de 3 à 5 atomes de carbone, notamment 4 ou 5 atomes de carbone,
   lequel tensioactif est choisi parmi
   - les tensioactifs anioniques,
   - les tensioactifs cationiques,
   - les tensioactifs amphotères,
   - les tensioactifs non-ioniques.
Selon un autre mode de réalisation avantageux,
La présente description concerne des microémulsions de type huile/eau consistant en,
- 80% à 94% d'eau,
- 1% à 15%, en particulier 3% à 10%, et notamment 5% à 10%, notamment environ 10%, de substances hydrophobes, en particulier de composition parfumante,
- 5% à 15%, notamment 5% à 10%, de plusieurs solvo-surfactifs volatils,
- 0,1% à 2% d'un ou plusieurs tensioactifs, de 0,2% à 2% d'un ou plusieurs tensioactifs, de préférence de 0,5 à 2% d'un ou plusieurs tensioactifs, en pourcentage massique,
le mélange de solvo-surfactif et de tensioactif ne contenant pas plus de 2% de composés non-volatils selon l'invention,
lequel solvo-surfactif est un composé amphiphile choisi parmi au moins un parmi
▪ les alcools gras polyéthoxylés CᵢEⱼ à chaîne carbonée linéaire, de formule CH₃-(CH₂)ᵢ₋₁-(O-CH₂-CH₂)ⱼ-OH,
   ou les alcools gras polyéthoxylés à chaîne ramifiée, de formule CᵢH₂ᵢ₊₁(OCH₂CH₂)ⱼOH,
   dans lesquelles
   - i représente le nombre d'atomes de carbone de la chaîne hydrocarbonée linéaire ou ramifiée et varie de varie de 3 à 8, de préférence de 3 à 6 et encore plus préférentiellement de 3 à 4,
   - j représente le nombre d'oxydes d'éthylène et varie de 1 à 4, notamment varie de 2 à 4, et de préférence est égal à 4.
   Selon un mode de réalisation préféré, j est égale à 4 quand i est égal à 6
   et au moins un dérivé choisi parmi les
▪ dérivés de glycérol CₖGly de formule 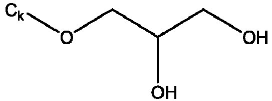 dans laquelle Cₖ représente
   - un groupe alkyle, linéaire ou ramifié, contenant de 1 à 6 atomes de carbone, en particulier de 3 à 5 atomes de carbone, notamment 4 ou 5 atomes de carbone,
   notamment C₄Gly de formule 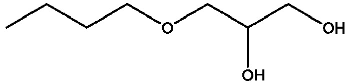 ou C₅Gly de formule 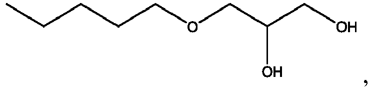 ou notamment le dérivé ramifié i-C₅Gly de formule 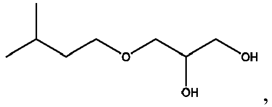
   - un groupe acyle, linéaire ou ramifié, contenant de 2 à 6 atomes de carbone, en particulier de 3 à 5 atomes de carbone, notamment 4 ou 5 atomes de carbone, notamment le pentanoate de glycéryle de formule 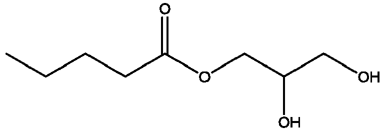
▪ et les dérivés d'isosorbide, IsosorbideCₘ₁ et IsosorbideCₘ₂, les dérivés d'isomannide, IsomannideCₘ₃, les dérivés d'isoidide, IsoidideCₘ₄ de formules 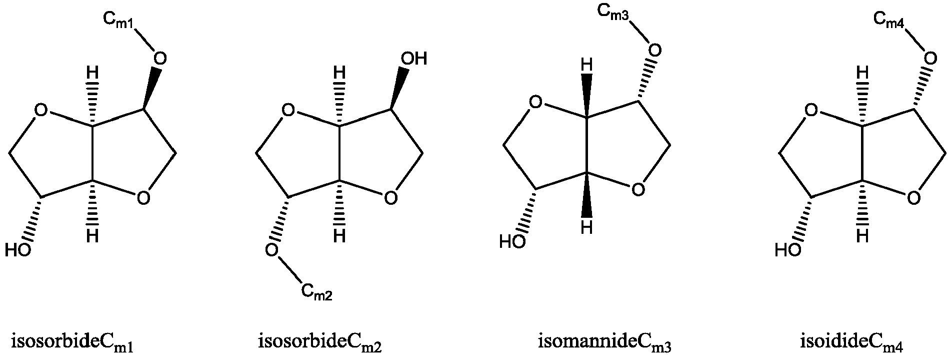 dans lesquelles Cₘ₁, Cₘ₂, Cₘ₃ et Cₘ₄ représentent
   - un groupe alkyle, linéaire ou ramifié, contenant de 1 à 6 atomes de carbone, en particulier de 3 à 5 atomes de carbone, notamment 4 ou 5 atomes de carbone,
   - un groupe acyle, linéaire ou ramifié, contenant de 2 à 6 atomes de carbone, en particulier de 3 à 5 atomes de carbone, notamment 4 ou 5 atomes de carbone,
   lequel tensioactif est choisi parmi
   - les tensioactifs anioniques,
   - les tensioactifs cationiques,
   - les tensioactifs amphotères,
   - les tensioactifs non-ioniques.

Selon un mode de réalisation particulier, la présente description concerne des microémulsions de type huile/eau consistant en,
- 80% à 94% d'eau,
- 1% à 15% en particulier 3% à 10%, et notamment 5% à 10%, notamment environ 10%, de substances hydrophobes, en particulier de composition parfumante
- 5% à 15%, notamment 5% à 10%, d'un ou de plusieurs solvo-surfactifs volatils,
- et 0,1% à 2% d'un ou plusieurs tensioactifs, de préférence de 0,2% à 2% d'un ou plusieurs tensioactifs, et de façon encore plus avantageuse de 0,5 à 2% d'un ou plusieurs tensioactifs, en pourcentage massique
le mélange de solvo-surfactif et de tensioactif ne contenant pas plus de 2% de composés non-volatils, selon l'invention
lequel solvo-surfactif est un composé amphiphile choisi parmi :
▪ les dérivés de glycérol CₖGly de formule 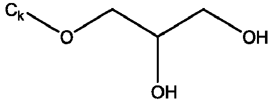 dans laquelle Cₖ représente
   - un groupe alkyle, linéaire ou ramifié, contenant de **1** à 6 atomes de carbone, en particulier de 3 à 5 atomes de carbone, notamment 4 ou 5 atomes de carbone, notamment C₄Gly de formule 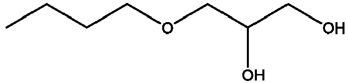 ou C₅Gly de formule 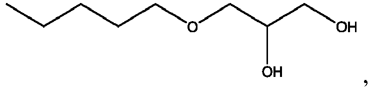 ou notamment le dérivé ramifié i-C₅Gly de formule 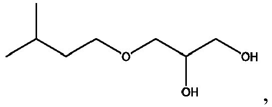
   - un groupe acyle, linéaire ou ramifié, contenant de 2 à 6 atomes de carbone, en particulier de 3 à 5 atomes de carbone, notamment 4 ou 5 atomes de carbone, notamment le pentanoate de glycéryle de formule 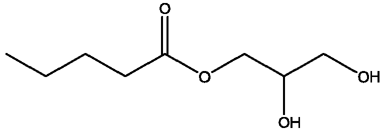
▪ les dérivés d'isosorbide, IsosorbideCₘ₁ et IsosorbideCₘ₂, les dérivés d'isomannide, IsomannideCₘ₃, les dérivés d'isoidide, IsoidideCₘ₄ de formules 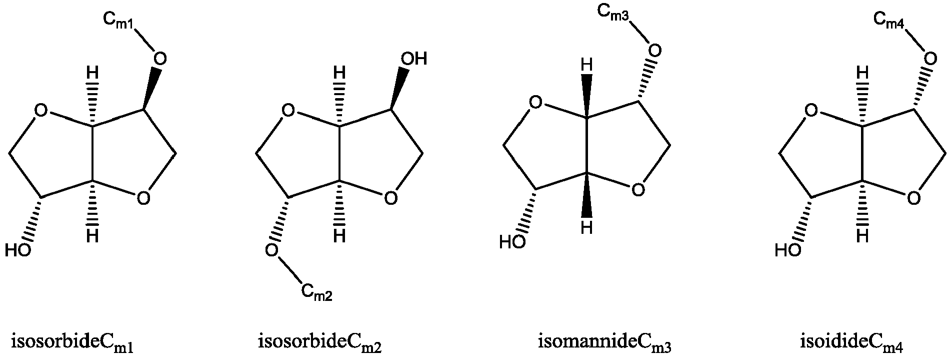 dans lesquelles Cₘ₁, Cₘ₂, Cₘ₃ et Cₘ₄ représentent
   - un groupe alkyle, linéaire ou ramifié, contenant de 1 à 6 atomes de carbone, en particulier de 3 à 5 atomes de carbone, notamment 4 ou 5 atomes de carbone,
   - un groupe acyle, linéaire ou ramifié, contenant de 2 à 6 atomes de carbone, en particulier de 3 à 5 atomes de carbone, notamment 4 ou 5 atomes de carbone,
   lequel tensioactif est choisi parmi
   - les tensioactifs anioniques,
   - les tensioactifs cationiques,
   - les tensioactifs amphotères,
   - les tensioactifs non-ioniques.

Par « **tensioactif** », on désigne un composé de nature amphiphile, non volatil, comportant une partie hydrophile et polaire, et une partie hydrophobe apolaire. Un tensioactif abaisse la tension superficielle des solutions aqueuses et diminue la tension interfaciale entre l'eau et un liquide organique non-miscible. Il permet ainsi de solubiliser deux phases non-miscibles, telles que l'eau et l'huile, en interagissant par sa partie polaire avec l'eau et par sa partie apolaire avec l'huile. Ils sont choisis parmi les quatre catégories de tensioactifs:
- anioniques: la partie hydrophile est chargée négativement,
- cationiques: la partie hydrophile est chargée positivement,
- amphotères ou zwitterioniques: la partie hydrophile comporte une charge positive et une charge négative, la charge globale étant nulle,
- non ioniques : le composé ne comporte aucune charge.
Le tensioactif est désigné par le terme **"booster"** dans les exemples.

La quantité de tensioactif(s) dans les microémulsions selon l'invention peut être égale à 0 et n'est pas supérieure à 2 % en pourcentage massique. La quantité de tensioactif(s) dans les microémulsions selon l'invention, avantageusement, n'est pas inférieure à 0,5 % en pourcentage massique, et plus particulièrement est comprise entre 0,2% et 2%, de préférence entre 0,95% et 1,90% ou encore entre 0,90% et 1,20%, en pourcentage massique.

Par « **microémulsion** », on désigne un système liquide monophasique contenant de l'huile, de l'eau ainsi que un ou plusieurs tensioactifs, ces composants s'organisant sous forme de microdomaines aqueux et huileux. Ces systèmes se forment spontanément par simple mélangeage des ingrédients et sont thermodynamiquement stables. Il est souhaitable que les microdomaines contenant les substances hydrophobes soient de petite taille (diamètre inférieur à 100 nm) pour que la composition ait un aspect transparent ou tout au moins translucide. On s'attachera donc à produire des microémulsions de diamètre compris de 2 à 100 nm répondant à ce critère d'aspect transparent, plutôt que des macroémulsions de diamètre supérieur à 0,1 µm.

Ces microémulsions sont donc invisibles à l'oeil nu et au microscope optique. Elles sont transparentes, contrairement aux émulsions, ce qui est une propriété recherchée notamment pour les compositions parfumantes.

Par « **microémulsion de type huile/eau** », on désigne les microémulsions de l'invention, dans lesquelles des gouttelettes d'huile sont dispersées dans une phase aqueuse, l'interface huile/eau étant stabilisée par des composés à propriétés tensioactives.

Par « **substance hydrophobe** », on désigne un corps pur ou un mélange insoluble ou très peu soluble dans l'eau, par nature. Une méthode possible pour déterminer l'hydrophobicité des substances est de mesurer leur solubilité dans différents solvants ou le temps de rétention sur colonne chromatographique (par chromatographie en phase liquide à haute performance HPLC) de la dite substance hydrophobe.

Les substances hydrophobes selon l'invention sont par exemple des huiles, et plus particulièrement des huiles essentielles, et de préférence des huiles essentielles utilisées dans les parfums. Selon un mode de réalisation préféré, on entend par substance hydrophobe, une substance parfumante ou composition parfumante.

Dans le terme **"solvo-surfactif", "solvo"** provient de **"solvant"** et « **surfactif** » provient du terme anglo-saxon « **surfactant** », équivalent au terme français de « **tensioactif ».** Par « **solvo-surfactif** », on désigne donc des composés amphiphiles, cumulant certaines propriétés des tensioactifs, notamment la diminution des tensions superficielle eau/air et interfaciales huile/eau, la capacité d'auto-association dans l'eau, et certaines propriétés des solvants, notamment la capacité à s'évaporer sans laisser de résidus.

Les solvo-surfactifs de l'invention peuvent être des alcools gras polyéthoxylés à chaîne carbonée courte, linéaire ou ramifiée, de formule CH₃-(CH₂)ᵢ₋₁-(O-CH₂-CH₂)ⱼ-OH ou de formule CᵢH₂ᵢ₊₁(OCH₂CH₂)ⱼOH, désignés par la formule générale CiEj. Les solvo-surfactifs de l'invention peuvent aussi être des dérivés du glycérol désignés par CiGly, en particulier des monoéthers ou des monoesters de glycérol, mais aussi des dérivés d'isosorbide, d'isomannide ou d'isoidide, en particulier des monoéthers ou des monoesters d'isosorbide, d'isomannide ou d'isoidide. Les solvo-surfactifs de l'invention ont notamment pour formule, ou sont choisis parmi C₄E₁, C₅E₁, C₅E₂, C₅E₃, C₆E₂, C₆E₃, C₆E₄, C₈E₂, C₄Gly, C₅Gly, IsosorbideC₄, IsosorbideC₅, et leur mélange. De façon avantageuse, les solvo-surfactifs de l'invention ont notamment pour formule ou sont choisis parmi C₄E₁, C₅E₁, C₅E₂, C₅E₃, C₆E₂, C₆E₃, C₆E₄, C₄Gly, C₅Gly, IsosorbideC₄, IsosorbideC₅, et leur mélange, de façon encore plus avantageuse les solvo-surfactifs de l'invention ont pour formule C₄E₁, C₅E₁, C₅E₂, C₅E₃, C₄Gly, C₅Gly, IsosorbideC₄, IsosorbideC_{5,}, ou encore C₆E₄, C₄Gly, C₅Gly, IsosorbideC₄, IsosorbideC₅ et leur mélange.

Dans les microémulsions selon l'invention, les solvo-surfactifs sont de façon avantageuse des composés amphiphiles choisis parmi C₅E₂, C₆E₂, C₆E₃ ou C₆E₄.

La quantité de solvosurfactif(s) en combinaison avec un ou plusieurs tensioactifs selon l'invention, de préférence de 0,5% à 2% un ou plusieurs tensioactifs selon l'invention, dans les microémulsions selon l'invention, est comprise entre 5% et 15% en pourcentage massique. Avantageusement, la quantité de tensioactif(s) dans les microémulsions selon l'invention, est comprise entre 5,5 et 12,5%,

Plus avantageusement, la quantité de solvosurfactif(s) dans les microémulsions selon l'invention, est comprise entre 8% et 10% lorsque la quantité de tensioactif est comprise entre 0,5% et 2 %, pour une quantité de parfum inférieure ou égale à 5%, d'environ 5 à 6 %. Un des avantages de ces formulations est de conserver leur stabilité et une qualité de fragrance stable en fonction du temps et pendant au moins 1 an, de préférence pendant 2 ans.

Les critères techniques de la qualité d'une fragrance sont la diffusion ou capacité d'une fragrance à se répandre dans l'air ambiant, la substantivité ou capacité d'une composition parfumée à maintenir, après application, un seuil de perception olfactif significatif dans le temps, la linéarité ou la capacité d'une composition à maintenir, une fois appliquée, sa forme olfactive dans le temps, la stabilité ou capacité d'une composition à ne pas subir d'altérations endogènes ou exogènes, qui pourraient modifier sa forme olfactive, l'innocuité qui est la faculté d'une composition à ne pas produire d'effets indésirables une fois appliquée sur la peau de l'utilisateur., un impact significatif dans l'esthétique des parfums.

Les compositions selon l'invention ont une qualité de fragrance stable pendant au moins deux ans et de préférence pendant au moins cinq ans.

Les composés solvo-surfactifs de l'invention peuvent être des composés « COV », (Composés Organiques Volatils). Plusieurs définitions de "COV" existent car plusieurs critères peuvent être utilisés pour les définir, notamment la pression de vapeur saturante (pression de la phase gazeuse du composé se trouvant en équilibre au-dessus de sa phase liquide, à une température donnée) ou la température d'ébullition (température à laquelle le changement d'état liquide/gaz a lieu) dudit composé. (Observatoire régional de santé d'Ile de France, Isabelle GREMY et al, les composés organiques volatils, décembre 2007).
- Ainsi, dans la réglementation américaine (Environmental Protection Agency), un COV est un composé organique dont la température d'ébullition est inférieure à 250°C sous une pression de 1 bar.
- Dans la réglementation européenne,
   - la directive 1999/13/CE (11 mars 1999, directive relative à la réduction des émissions de composés organiques volatils dues à l'utilisation de solvants organiques dans certaines activités et installations) définit les COV comme des "composés organiques ayant une pression de vapeur de 0,010 kPa ou plus, à une température de 293,15 K";
   - la directive 2004/42/CE (29 mai 2006, directive relative à la réduction des émissions de composés organiques volatils dues à l'utilisation de solvants organiques dans certains vernis et peintures et dans les produits de retouche de véhicules) regroupe les COV d'après leur "point d'ébullition inférieur à 250°C à la pression standard de 101,3 kPa";
   - la norme NF ISO 16000-6 (OQAI, Observatoire de la qualité de l'air intérieur, 2001) définit les COV "selon leur température d'ébullition et distingue, d'après la classification adoptée par l'OMS en 1989, les composés organiques très volatils, volatils et semi-volatils ".

Le but de ces directives est de permettre la diminution des émissions des composés organiques volatils, considérés comme nocifs pour l'environnement et la santé de l'homme. Ainsi, les alcools comportant de 1 à 10 atomes de carbone notamment, les alcools polyhydroxylés comportant de 1 à 6 atomes de carbone notamment, ou encore le propylène glycol, répondent à ces définitions.

Un composé non-volatil selon l'invention ne répond à aucune définition citée ci-dessus.

Le "caractère volatil", en particulier des solvo-surfactifs de l'invention, est défini et comparé à celui de substances parfumantes ou de solvants usuels en parfumerie par des mesures en analyse thermogravimétrique (Figure 1).

Dans l'invention, les domaines de "volatilité COV" et de "volatilité non-COV" ont été définis en comparaison à des matières premières couramment utilisées dans les parfums, soit en tant que substances parfumantes soit en tant que solvant de parfumerie; un composé volatil s'évapore plus vite que les molécules de parfums ou les solvants utilisés en parfumerie.

Dans l'invention, on définit les composés, en particulier les "composés volatils" par leur température d'ébullition inférieure à 250 °C à pression atmosphérique tandis que l'on définit les composés non-volatils par leur température d'ébullition supérieure à 250 °C à pression atmosphérique. Le critère pris en compte ici, est donc la température d'ébullition du composé testé, la technique de thermogravimétrie consistant, en effet, à mesurer la variation de masse d'un échantillon de ce composé en fonction de la température à laquelle il est chauffé (figure 1, représentation de la température pour laquelle 98% en masse de l'échantillon sont évaporés (T_{98% ATG}) en fonction du nombre d'atomes de carbone du composé, après 40 minutes de chauffage).
De plus, les composés volatils selon l'invention sont classés en trois catégories définies de la façon suivante:
- "COV très volatils": leur température d'ébullition à pression atmosphérique est inférieure à 70°C;
- "COV volatils": leur température d'ébullition à pression atmosphérique est comprise de 70°C à 160°C : cas des solvo-surfactifs de l'invention choisis parmi C₄E₁, C₅E₁, C₅E₂, C₆E₂,
- "COV semi-volatils": leur température d'ébullition à pression atmosphérique est comprise de 160°C à 250°C, cas des solvo-surfactifs de l'invention choisis parmi C₅E₃, C₆E₃, C₆E₄, C₈E₂, C₄Gly et C₅Gly.

Un des avantages de l'invention consiste à judicieusement associer de 5 à 15 % d'un parfum à un faible pourcentage de solvo-surfactif (de 5 à 15%) en combinaison avec un faible pourcentage de tensioactif (de 0,1% à 2% ou de 0,2% à 2%, ou avantageusement de 0,5 % à 2 % de tensioactif), le tensioactif pouvant être qualifié de **"booster".** En effet, cette association, par effet de synergie, permet de diminuer la quantité de solvo-surfactif nécessaire à la solubilisation totale de la substance hydrophobe de la microémulsion de l'invention, tout en obtenant un liquide stable et d'aspect transparent.

Par conséquent, l'invention permet de fournir des compositions comprenant une quantité de substances non volatiles selon l'invention (ayant une température d'ébullition supérieure à 250 °C à pression atmosphérique) inférieure à 2% dans le mélange tensioactif solvosurfactif et avantageusement elle permet de fournir des compositions sans aucune substance non volatile selon l'invention dans le mélange tensioactif solvosurfactif.

Selon un mode de réalisation particulier l'invention permet de fournir des compositions comprenant une quantité de substances non volatiles selon l'invention (ayant une température d'ébullition supérieure à 250 °C à pression atmosphérique) inférieure à 2% dans le mélange tensioactif solvosurfactif et de réduire l'utilisation de composés organiques volatiles (COV).

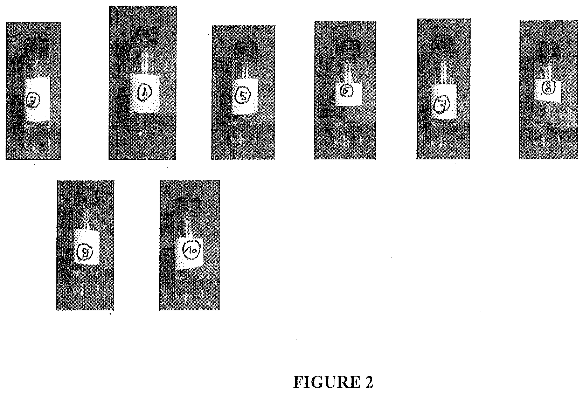

L'effet de ces combinaisons est d'obtenir des compositions ne laissant pas de résidus (figures 2-6) (détectable à l'oeil nu) tout en limitant l'utilisation de composés organiques volatiles (COV) susceptible d'induire des effets indésirables chez l'utilisateur et/ou sur l'environnement..

On entend par « sans aucune substance » une composition comprenant une quantité indétectable de la dite substance par les moyens connus de l'homme du métier, comme la chromatographie en phase liquide (HPLC), soit une quantité inférieure à 0,001% en pourcentage massique.

Dans l'expression **"le mélange de solvo-surfactif et de tensioactif ne contenant pas plus de 2% de composés non-volatils",** le pourcentage est massique. Selon un mode de réalisation préféré, au plus, 2% ne sont pas volatils selon la définition ci-dessus, par rapport à la masse totale de la composition contenant la substance hydrophobe, l'eau, le solvo-surfactif et le tensioactif.

La présente invention porte sur des microémulsions comprenant le moins possible de ces substances non volatiles, dans la composition totale. On entend par "le mélange de solvo-surfactif(s) et de tensioactif(s) ne contenant pas plus de 2% de composés non-volatils, des microémulsions comprenant pas plus de 2% de composés non-volatils par rapport à la masse totale de la composition.

Selon un mode de réalisation préféré, l'invention permet de fournir des compositions sans aucune substance non volatile selon l'invention dans toute la composition.

Un des avantages de la présente invention est donc de fournir des compositions de parfum dans lesquelles il est possible de n'utiliser aucune substance (ou des quantités inférieures à 0,001% en pourcentage massique, ou des quantités indétectables) ayant une température d'ébullition supérieure à 250 °C à pression atmosphérique. Ces microémulsions sont stables, ne laissent pas de traces et présentent un risque d'induire des effets secondaires, comme des allergies, très réduit.

De façon encore plus avantageuse, la masse totale de la composition ne contient pas plus de 15%, pas plus de 7,5%, ou pas plus de 5% de **COV semi-volatils selon l'invention, ou** pas plus de 15% de substances ayant une température d'ébullition à pression atmosphérique comprise de 70°C à 160°C.

De façon évidente, une grande quantité de tensioactif est un désavantage car après évaporation des substances hydrophobes et de l'eau, les compositions obtenues peuvent laisser des résidus collants, irritants, allergisants, visibles (taches) sur la peau, les cheveux, le textile. Les compositions de l'invention permettent ainsi d'éviter ces effets indésirables.

De façon avantageuse, le mélange de solvo-surfactif et de tensioactif des microémulsions de l'invention ne contient pas de composés non-volatils.

Le mélange est dans ce cas évaporé en totalité après son application.

De façon avantageuse, les microémulsions selon l'invention, sont dépourvues
- d'alcools linéaires ou ramifiés, comprenant de 1 à 7 atomes de carbone,
- de glycols et de polyéthylènes glycol (PEG),
- de polyols tels que les dihydroxyalcanes,
- de sucres ou leurs dérivés.

Il n'est pas évident de préparer les microémulsions de l'invention sans alcool et notamment sans éthanol, celui-ci étant un excellent solvant de solubilisation de substances hydrophobes, permettant d'obtenir des solutions transparentes, homogènes et stables. L'éthanol présente cependant l'inconvénient d'être un composé COV, et l'inconvénient d'être prohibé par certaines communautés religieuses. Un autre avantage de l'invention est donc l'éviction d'éthanol et, plus largement des alcools contenant jusqu'à 7 atomes de carbone, et l'éviction des éthers d'éthylène glycol et de propylène glycol et de leurs acétates, des microémulsions de l'invention.

De façon plus avantageuse, les microémulsions de l'invention contiennent des composés volatils et de façon encore plus avantageuse contient des composés volatils et moins de 10% de composés organiques volatils ou très volatils.

Selon un mode de réalisation préféré, la présente description a pour objet une microémulsion de type huile/eau consistant en,
- 80% à 94% d'eau,
- 1% à 15% de substance hydrophobes, ou de composition parfumante en particulier 3% à 10%, notamment environ 10%,
- 5% à 15%, notamment 5% à 10%, d'un ou de plusieurs solvo-surfactifs volatils, tels que définis ci-dessus,
- et 0,1% à 2% d'un ou plusieurs tensioactifs, en pourcentage massique, tels que définis ci-dessus,
et dans laquelle la quantité de solvant ou ingrédient de solubilisation de substances hydrophobes est comprise entre 0,001% et 0,01% en pourcentage massique, ou indétectable par HPLC ou chromatographie en phase gazeuse.

On entend par « pur » un composé présentant un 0,01% d'impuretés, de préférence 0,001% d'impuretés. On entend par un composé « dépourvu de » un composé présentant 0,001% de composé dont il est dépourvu, de préférence présentant 0,01% de composé dont il est dépourvu.

Les **"polyéthylènes glycols"** (PEG), sont des composés largement utilisés en raison de leur solubilité dans l'eau et dans les solvants organiques. Ils sont soupçonnés de provoquer des irritations cutanées, de faciliter la pénétration par la peau de substances lesquelles peuvent être nocives, et d'être impliqués dans des atteintes du système nerveux central. Par conséquent, il est souhaitable de s'affranchir de tels composés dans les parfums.

De façon avantageuse, les microémulsions selon l'invention, sont également dépourvues
- d'ingrédients de solubilisation choisis parmi les sels d'ammonium qui ne sont pas des tensioactifs,
- d'ingrédients de solubilisation choisis parmi les sels de sulfonates de naphtalène, de toluène ou de xylène,
- de sel alcalin et/ou de sel alcalino-terreux,
- de tensioactifs tels que les SPAN ou leurs dérivés, et tels que les TWEEN,
- de tensioactifs de la famille des alkylpolyglycosides.

Par **"ingrédient de solubilisation",** on désigne un composé chimique hydrotrope qui aide à la solubilisation, notamment d'une substance hydrophobe dans de l'eau, sans être classé chez les composés tensioactifs. Les microémulsions de l'invention ne contiennent donc aucun des ingrédients suivants, (ou des quantités indétectables par tout moyen approprié connu de l'homme du métier) considérés comme des ingrédients de solubilisation : sels d'ammonium d'acide pyridine carboxyliques, d'acide pyrrolidone carboxyliques, tel que le composé commercial "Ajidew NL-50", de proline, d'acide benzoïque et de ses dérivés hydroxylés et aminés, d'acide lactique, d'acide succinique, d'acide oxalique, d'acide citrique, d'acide tartrique, d'acides naphtalène-sulfoniques éventuellement substitués.

Par "sel **minéral alcalin et/ou sel minéral alcalino/terreux",** on désigne notamment NaCl, KCl, CaCl₂, MgCl₂, exclus de la composition des microémulsions de l'invention.

Les **"SPAN ou leurs dérivés et les TWEEN"** sont constitués d'esters gras de sorbitol, encore appelés **"polysorbates",** tels que le monooléate de sorbitan (SPAN 80), le monostéarate de sorbitan (SPAN 60), le monopalmitate de sorbitan (SPAN 40), le monolaurate de sorbitan (SPAN 20), le trioléate de sorbitan (SPAN 85), et leurs dérivés polyéthoxylés **"Tween".**

Les **"alkylglycosides"** sont des polyosides dérivés de sucres. Les sulfonates aromatiques tels que les sulfonates de sodium du mono ou diméthyle naphtalène, ou le sulfonate de sodium du xylène ne sont pas présents dans les microémulsions de l'invention.

Selon un mode de réalisation préféré, dans les microémulsions de l'invention, la « **substance hydrophobe** » est une substance parfumante choisie parmi un terpène, un terpénoïde, une huile essentielle, ou un composé synthétique présentant des propriétés odoriférantes, notamment choisi parmi les aldéhydes, les esters, les cétones, les ionones, les éthers, ladite substance parfumante pouvant être une composition parfumante complexe, contenant un mélange de plusieurs terpènes, terpénoïdes, huiles essentielles, ou plusieurs composés odoriférants synthétiques purs ou en solution, le pourcentage massique de ladite substance ou composition parfumante étant compris de 1% à 10%, et notamment de 3% à 10%, et étant en particulier environ égal à 5% ou environ égal à 10%.
Les substances hydrophobes selon l'invention sont par exemple des huiles, et plus particulièrement des huiles essentielles, et de préférence des huiles essentielles utilisées dans les parfums.

Par « odeur » on entend une émanation transmise par l'air perçue par l'appareil olfactif, en particulier l'appareil olfactif humain.

Par « **substance (ou composé) odoriférante** » on entend une substance détectable olfactivement par un sujet et/ou par olfactométrie, selon des principes connus de l'homme du métier. Un exemple de méthode permettant la détection d'une substance odoriférante est décrit dans le document EP 0003088. D'autres techniques de détection d'une substance odoriférante sont applicables comme les techniques de chromatographie en phase gazeuse, de spectroscopie de masse ou encore d'analyse par absorption infrarouge.

On entend aussi par substance odoriférante une substance qui répand une odeur, de préférence agréable- pour au moins 20% de personnes -, en particulier un parfum.

Par **"substance parfumante",** on désigne un composé pur ou un mélange de composés, le(s)dit(s) composé(s) possédant des propriétés odoriférantes utilisées en parfumerie.

Par **"terpènes",** on désigne des hydrocarbures dont l'élément de base est l'isoprène, leur formule brute comportant un nombre de carbones multiple de 5, en particulier des terpènes contenant notamment 10 ou 15 atomes de carbone, utilisés en parfumerie.

Par "**terpènoïdes**", on désigne des dérivés des terpènes, par exemple des alcools, des phénols, des cétones, des aldéhydes, des esters, des éthers.

Terpènes et terpénoïdes sont contenus dans les **"huiles essentielles",** désignant le liquide concentré, très souvent odoriférant, volatil, produit par les plantes. Ces huiles essentielles sont le plus souvent extraites des organes de la plante par hydrodistillation notamment, mais les constituants de ces huiles sont largement synthétisés par l'industrie.

La liste suivante de composés odoriférants, fournie à titre d'illustration, n'est en aucun cas limitative :
terpènes: pinènes, camphènes, limonène, cadinène, carène, caryophyllène,
alcools : linalol, géraniol, menthol, citronellol,
cétones: menthone, carvone, béta-ionone, thuyone, camphre, cyclopentadécanone,
aldéhyde : citral, citrannal, citronellal, aldéhyde cinnamique, lilial,
esters: acétate de linalyle, acétate de menthyle, acétate de géranyle, succinate de géranyle,
phénols : thymol, carvacrol, eugénol, isoeugénol,
éthers: anéthol, eucalyptol, cinéol, oxyde de rose.

Les huiles essentielles peuvent être les huiles d'ylang-ylang, de bergamote, d'eucalyptus, de lavande, de lavandin, de lemon grass, de patchouli, de menthe poivrée, de pin, de rose, de coriandre, de Shiu, de sauge, de géranium, de palmarosa, de Litsea Cubeba, de citron, de citronnelle, de fleur d'oranger, de pamplemousse, de lime, de mandarine, de tangerine, d'orange, de cajeput, de camphre, de romarin, d'anis vert, d'anis étoilé, de fenouil, de basilic, d'estragon, de girofle, de piment, de thym, de sassafras, d'absinthe, d'armoise, de cèdre, d'hysope, de tagetes, de rue, d'elemi, de galbanum, de baies de genièvre, de cabreuva, de bois de gaiac, de bois de santal, de vétiver, d'ambrette, d'angélique, de rhizome d'iris, de carotte, de céleri, de cumin, de livèche, de persil, de cannelle, de cardamome, de gingembre, de noix de muscade, de poivre, d'oliban, de myrrhe, de baume du Pérou, de styrax, de buchu, de camomille ou de ciste (Jean Garnero, "Huiles essentielles", *Techniques de l'ingénieur,* Traité constantes physico-chimiques, K-345).

De façon avantageuse, les microémulsions de l'invention sont constituées d'environ 5% de substance parfumante, environ 85% d'eau, de 5% à 10% de solvo-surfactif et d'environ 1% de tensioactif.

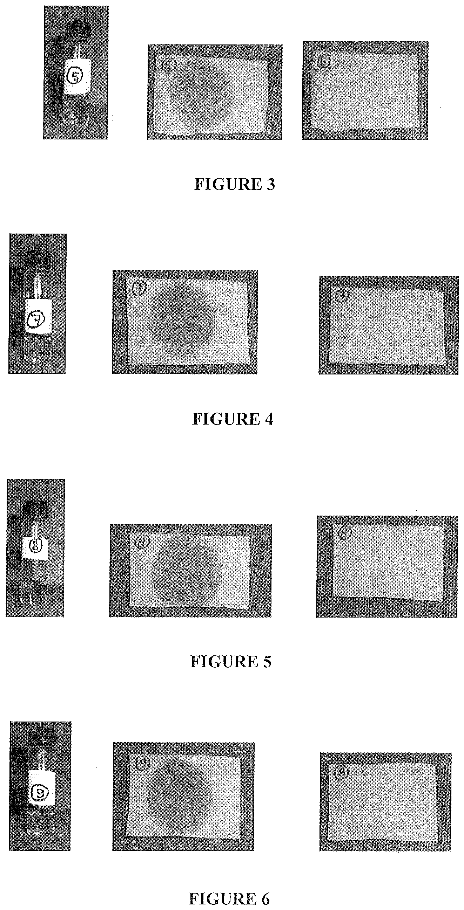

Selon une composition avantageuse, les microémulsions de l'invention contiennent environ 5 % de substances parfumantes et ne contiennent que très peu de tensioactifs, environ 1 %, ce qui permet d'éviter le caractère collant et « tachant » des résidus non volatils subsistant éventuellement sur la peau et/ou les tissus.
L'absence de caractère "tachant" peut être mis en évidence par des photographies de tissus prises après 4 ou 5 heures à 20°C (figure 3-6).

De façon particulièrement avantageuse, les microémulsions de l'invention sont constituées d'environ 10% de substance parfumante, d'environ 80% d'eau, de 8% à 10% de solvo-surfactif et de 0,1% à 2% de tensioactif, et notamment d'environ 1% de tensioactif. Selon une composition particulièrement avantageuse, les microémulsions de l'invention contiennent jusqu'à environ 10 % de substances parfumantes et ne contiennent que très peu de tensioactifs, pourcentage toujours inférieur à 2 %.

Dans les microémulsions de la présente description, selon un mode de réalisation particulier, les solvo-surfactifs sont des composés amphiphiles choisis parmi les alcools gras polyéthoxylés CᵢEⱼ à chaîne carbonée linéaire, de formule CH₃-(CH₂)ᵢ₋₁-(O-CH₂-CH₂)ⱼ-OH, ou les alcools gras polyéthoxylés à chaîne ramifiée, de formule CᵢH₂ᵢ₊₁(OCH₂CH₂)ⱼOH, dans lesquelles
- i représente le nombre d'atomes de carbone de la chaîne hydrocarbonée linéaire ou ramifiée et varie de 3 à 8, notamment varie de 4 à 6,
- j représente le nombre d'oxydes d'éthylène et varie de 1 à 4, notamment varie de 2 à 4,
et sont en particulier C₅E₂, C₆E₂, C₆E₃ ou C₆E₄.

Par " **CⱼEⱼ**", on désigne des alcools gras polyéthoxylés à chaîne carbonée courte de formule CH₃-(CH₂)ᵢ₋₁-(O-CH₂-CH₂)ⱼ-OH si la chaîne alkyle est linéaire, ou de formule CᵢH₂ᵢ₊₁(OCH₂CH₂)ⱼOH, si la chaîne alkyle est ramifiée. Ils peuvent être utilisés purs, et seront alors en particulier
- C₅E₂ de formule CH₃-(CH₂)₄-(O-CH₂-CH₂)₂-OH (i=5 et j=2),
- C₆E₂ de formule CH₃-(CH₂)₅-(O-CH₂-CH₂)₂-OH (i=6 et j=2),
   ou C₆E₃ de formule CH₃-(CH₂)₅-(O-CH₂-CH₂)₃-OH (i=6 et j=3, ou C₆E₄ de formule CH₃-(CH₂)₅-(O-CH₂-CH₂)₄-OH (i=6 et j=4) si la chaîne est linéaire,
- ou, si la chaîne est ramifiée, les composés de formule CᵢH₂ᵢ₊₁(OCH₂CH₂)ⱼOH, notamment les composés de formule : 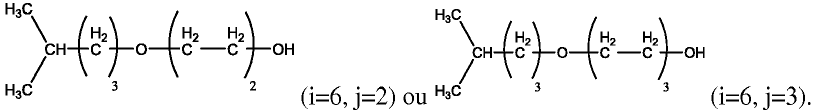 C₆E₂ est un composé volatil COV; C₆E₃ et C₆E₄ sont des composés semi volatils non-COV (figure 1).

Dans les microémulsions de la présente description, selon un mode de réalisation particulier, les solvo-surfactifs sont utilisés purs et sont des dérivés de glycérol CₖGly de formule 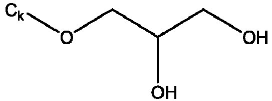 dans laquelle Cₖ représente
- un groupe alkyle, linéaire ou ramifié, contenant de 1 à 6 atomes de carbone, en particulier de 3 à 5 atomes de carbone, notamment 4 ou 5 atomes de carbone, notamment C₄Gly de formule 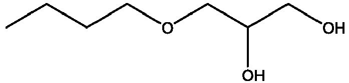 ou C₅Gly de formule 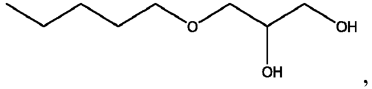 ou notamment le dérivé ramifié i-C₅Gly de formule 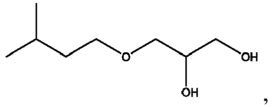
- un groupe acyle, linéaire ou ramifié, contenant de 2 à 6 atomes de carbone, en particulier de 3 à 5 atomes de carbone, notamment 4 ou 5 atomes de carbone, notamment le pentanoate de glycéryle de formule 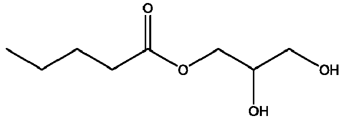
   - Si Cₖ est un groupe alkyle, ces composés sont des éthers de glycérol. Ils sont notamment représentés par les formules de C₄Gly et de C₅Gly si le groupe alkyle est linéaire,
      et représentés notamment par i-C₅Gly si le groupe alkyle est ramifié. C₄Gly, C₅Gly et i-C₅Gly sont des composés volatils non-COV (figure 1),
   - Si Cₖ est un groupe acyle, ces composés sont des esters de glycérol.

Les solvo-surfactifs sont utilisés purs, signifie qu'ils sont utilisés non dilués.

Les formules du dérivé ramifié i-C₅Gly et du pentanoate de glycéryle sont représentées à titre d'illustration mais ne sont en aucun cas limitatives.

Dans les microémulsions de la présente description, selon un mode de réalisation particulier, les solvo-surfactifs sont utilisés purs et sont des dérivés d'isosorbide, IsosorbideCₘ₁ et IsosorbideCₘ₂, les dérivés d'isomannide, IsomannideCₘ₃, les dérivés d'isoidide, IsoidideCₘ₄ de formules 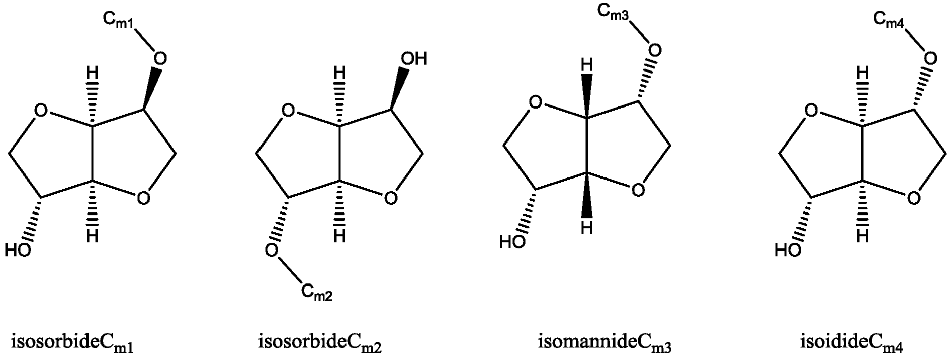 dans lesquelles Cₘ₁, Cₘ₂, Cₘ₃ et Cₘ₄ représentent
- un groupe alkyle, linéaire ou ramifié, contenant de 1 à 6 atomes de carbone, en particulier de 3 à 5 atomes de carbone, notamment 4 ou 5 atomes de carbone,
- un groupe acyle, linéaire ou ramifié, contenant de 2 à 6 atomes de carbone, en particulier de 3 à 5 atomes de carbones, notamment 4 ou 5 atomes de carbone.
   - Si Cₘ₁, Cₘ₂, Cₘ₃ ou Cₘ₄ représente un groupe alkyle, ces composés sont des éthers.
   - Si Cₘ₁, Cₘ₂, Cₘ₃ ou Cₘ₄ représente un groupe acyle, ces composés sont des esters.

Dans les microémulsions de la présente description, selon un mode de réalisation particulier, les solvo-surfactifs sont choisis parmi les alcools gras polyéthoxylés de formule CᵢEⱼ, les dérivés de glycérol de formule CₖGly et les dérivés d'isosorbide, IsosorbideCₘ₁ et IsosorbideCₘ₂, les dérivés d'isomannide, IsomannideCₘ₃, les dérivés d'isoidide, IsoidideCₘ₄, utilisés en mélange d'au moins deux composés solvo-surfactifs,
le mélange étant en particulier constitué d'au moins 2 CᵢEⱼ,
ou d'un CᵢEⱼ et au moins un dérivé de glycérol CₖGly,
ou d'un CᵢEⱼ et au moins un dérivé d'isosorbide, d'isomannide, ou d'isoidide,
ou d'un dérivé de glycérol CₖGly et au moins un dérivé d'isosorbide, d'isomannide, ou d'isoidide,
le mélange étant notamment constitué de C₆E₂ et de C₄Gly, dans les proportions de 1 pour 1 en volume.

Dans les microémulsions de l'invention, selon un mode de réalisation particulier, les solvo-surfactifs sont utilisés en mélange d'au moins deux composés solvo-surfactifs,
le mélange étant en particulier constitué d'au moins 2 deux CᵢEⱼ,
ou d'un CᵢEⱼ et au moins un dérivé de glycérol CₖGly de formule : 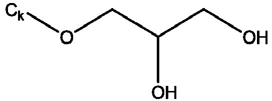 dans laquelle Cₖ représente
- un groupe alkyle, linéaire ou ramifié, contenant de 1 à 6 atomes de carbone, en particulier de 3 à 5 atomes de carbone, notamment 4 ou 5 atomes de carbone, notamment C₄Gly de formule 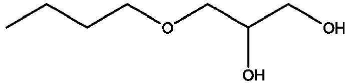 ou C₅Gly de formule 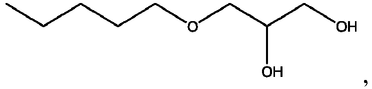 ou notamment le dérivé ramifié i-C₅Gly de formule 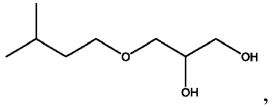
- un groupe acyle, linéaire ou ramifié, contenant de 2 à 6 atomes de carbone, en particulier de 3 à 5 atomes de carbone, notamment 4 ou 5 atomes de carbone, notamment le pentanoate de glycéryle de formule 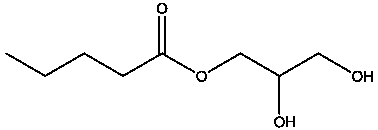 ou d'un CᵢEⱼ et au moins un dérivé d'isosorbide, d'isomannide, ou d'isoidide, le mélange étant notamment constitué de C₆E₂ et de C₄Gly, dans les proportions de 1 pour 1 en volume.

De façon avantageuse, les microémulsions de l'invention contiennent des tensioactifs anioniques et représentés par
- les alkylsulfonates, notamment le sulfosuccinate de dihexyle (DHS) de formule 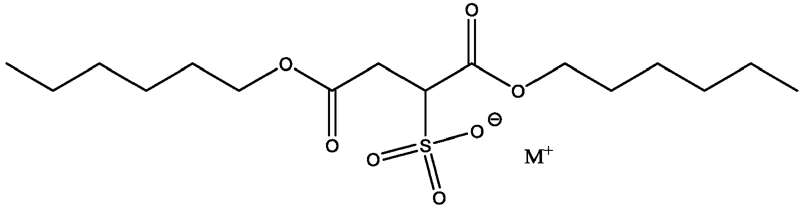 dans laquelle
   M⁺ représente Na⁺, K⁺, NH₄⁺, (HOCH₂CH₂)₃NH⁺,
   ou notamment le sulfosuccinate d'éthyl-2-hexyle (Aérosol OT® ou AOT de chez CYTEC) de formule 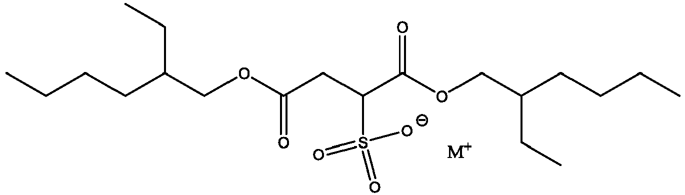 dans laquelle
   M⁺ représente Na⁺, K⁺, NH₄⁺, (HOCH₂CH₂)₃NH⁺,
- les alkylarylsulfonates de formule 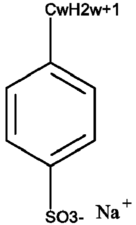 dans laquelle w est un entier compris de 8 à 12,
   en particulier l'isooctyl, l' isononyl et notamment l'isododécylbenzènesulfonate de sodium (SDBS) de formule 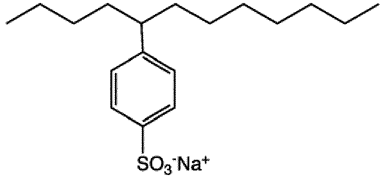
- les propoxysulfates, notamment les composés alfoterra® de formule 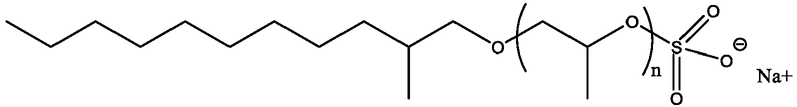 dans laquelle le nombre de motifs propoxylate n est compris de 4 à 8,
   □ n pouvant être notamment égal à 4 (alfoterra® 4S, Alf4S),
   □ n pouvant être notamment égal à 8 (alfoterra® 8S, Alf8S),
- ou les alkylsulfates, notamment les sels du sulfate de lauryle tel que le dodécyl sulfate de sodium (SDS) et les alkyléther sulfates de sodium tel que le lauryléther sulfate de sodium (LESNa),
- ou les sels d'acides gras de formule R-CO₂⁻ M⁺,
dans laquelle R représente une chaîne carbonée, linéaire ou ramifiée, saturée ou insaturée, contenant 8 à 18 atomes de carbone, et M⁺ représente un cation choisi parmi les ions Na⁺, K⁺, NH₄⁺, (HOCH₂CH₂)₃NH⁺,
notamment le sel d'acide oléique de formule CH₃(CH₂)₇CH=CH(CH₂)₇CO2⁻ M⁺ dans lequel M⁺ a les significations définies ci-dessus.

Par **"tensioactifs anioniques",** on désigne des composés chargés négativement en solution aqueuse. Ils sont largement utilisés dans les shampoings, les bains douches, pour leurs propriétés mouillantes et moussantes.

D'une autre façon avantageuse, les microémulsions de l'invention contiennent des tensioactifs cationiques sous forme de sels d'ammonium choisis parmi
- des sels de tétraalkylammonium, notamment des sels de dialkyldiméthylammonium de formule 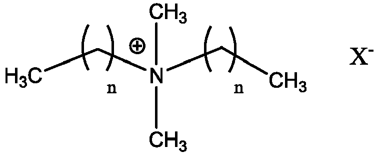 dans laquelle
   X⁻ représente un anion choisi parmi Cl⁻, Br⁻, I⁻ , CH₃COO⁻ ou lactate,
   n est le nombre de méthylène compris de 6 à 12,
   et représente notamment un sel de didécyl diméthylammonium [DiC₁₀][X] de formule 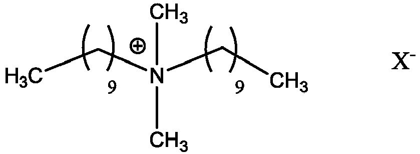 dans laquelle X⁻ a les significations désignées ci-dessus,
- des sels de trialkylammonium, en particulier des halogénures de trialkylammonium, notamment le bromure d'hexadécyl triméthylammonium (CTaBr) de formule H₃C(CH₂)₁₅N⁺(CH₃)₃,
- des sels de pyridinium, notamment le sel de cétylpyridinium de formule 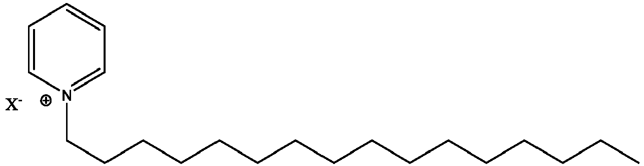 dans laquelle
   X⁻ a les significations désignées ci-dessus,
- des sels de benzalkonium notamment des sels de formule 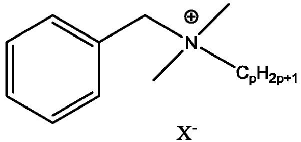 dans laquelle
   X⁻ a les significations désignées ci-dessus,
   p est compris de 8 à 18,
- des sels d'ammonium de la triéthanolamine de formule

   (HO-CH₂-CH₂)₃NH⁺ X⁻,

   dans laquelle
   X⁻ a les significations désignées ci-dessus.

Par **"tensioactifs cationiques",** on désigne des composés chargés positivement en solution aqueuse. Ils sont largement utilisés dans les produits de soins capillaires notamment. Ils possèdent des propriétés mouillantes, détergentes, émulsifiantes et éventuellement antiseptiques.

D'une autre façon avantageuse, les microémulsions de l'invention contiennent des tensioactifs amphotères choisis parmi
- les bétaïnes et représentent notamment la bétaïne de cocamidopropyle (CAPB) de formule, 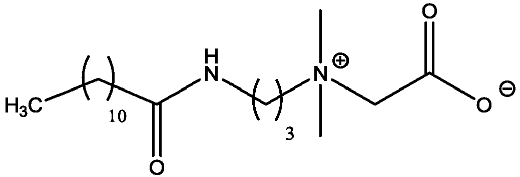
- ou les oxydes d'amine et représentent notamment l'oxyde de lauryldiméthylamine de formule 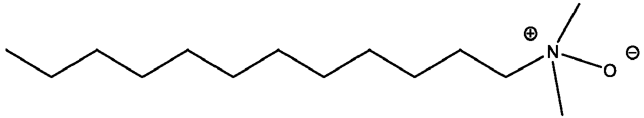 Par **"tensioactifs amphotères"** ou zwitterioniques, on désigne des composés chargés à la fois négativement par certaines fonctions, notamment carboxylates, et chargés positivement, notamment par des fonctions ammonium, en solution aqueuse. Ils s'adaptent donc au pH du milieu dans lequel ils se trouvent. Ils sont utilisés dans les shampoings, les bains douches, les bains moussants, pour leurs propriétés mouillantes, moussantes, antistatiques, adoucissantes et certains sont bactéricides.

D'une autre façon avantageuse, les microémulsions de l'invention contiennent des tensioactifs non-ioniques choisis parmi
- les alcanolamides, notamment le monoéthanolamide (cocamide, MEA) de formule 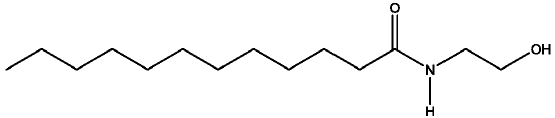 sous réserve que ledit alcanolamide ne soit pas un polyol,
- les alkylpolyglycosides (APG),
- les éthers de polyglycérol,
- ou les esters de polyglycérol.

Par **"tensioactifs non-ioniques",** on désigne des composés moléculaires, non-chargés en solution. Ils appartiennent à trois grandes catégories : les esters, les éthers et les amides.

Dans les microémulsions de l'invention, le rapport massique entre la substance parfumante définie ci-dessus et le ou les solvo-surfactif(s) varie de 0,5 à 1.

Dans les microémulsions de l'invention, le rapport massique entre la substance parfumante définie ci-dessus ou les tensioactif(s) varie de 5 à 100, notamment de 2,5 à 20.

De façon très avantageuse, les microémulsions de l'invention sont stables thermodynamiquement et ont un aspect transparent ou translucide (figure 2) et de façon encore plus avantageuse, les microémulsions de l'invention sont stables thermodynamiquement et ont un aspect transparent ou translucide pendant au moins un ou deux ans.

Les densités optiques (DO) dans l'UV et le visible des compositions parfumantes de l'invention ont été mesurées et comparées à celles correspondant à la substance parfumante en solution dans l'éthanol. Dans tous les cas, les spectres UV sont identiques et ne présentent aucune absorbance au-delà de 400 nm. En effet, le rapport des DO des microémulsions / solution éthanolique est égal à 1 pour λ > 400 nm.

Les microémulsions de l'invention sont stables à une température variant de 5°C à 55°C et notamment de 10°C à 40°C, pendant une durée d'au moins 5 ans dans ces gammes de température, notamment de 2 ans dans ces gammes de température.
Un avantage des microémulsions de l'invention est de pouvoir être conservées pendant des années, de deux à 10 ans, dans des conditions de température qui soient des conditions ordinaires, c'est-à-dire comprise entre 15 et 30°C.

De façon très avantageuse, les microémulsions de l'invention ne présentent ni caractère collant sur la peau, ni résidu visible sur un tissu.
L'absence de résidu est démontrée par les photos correspondant par exemple à la figure 3.

De façon particulière, les microémulsions de la présente description sont constituées
- d'environ 80% d'eau,
- d'environ 10% d'une substance parfumante choisie parmi un terpène, un terpénoïde, une huile essentielle, ou de façon générale un composé naturel ou synthétique présentant des propriétés odoriférantes, notamment choisi parmi les aldéhydes, les esters, les éthers, les cétones, les ionones, ladite substance parfumante n'étant pas un mélange,
- d'environ 10% de solvo-surfactif ,
- et d'environ 0,1% à 2% de tensioactif et notamment de 0,5%, 1% ou 2%.

De façon particulière, les microémulsions de la présente description sont constituées
- d'environ 80% d'eau,
- d'environ 10% d'une substance parfumante choisie parmi une huile essentielle,
- d'environ 10% de solvo-surfactif C₆E₄,
- et d'environ 0,1% à 2% de tensioactif et notamment de 0,5%, 1% ou 2%,

Les microémulsions de la présente description sont également constituées de :
- environ 80 à 85% d'eau,
- environ 5 à 10% d'une substance parfumante contenant un mélange de plusieurs substances choisies parmi les terpènes, les terpénoïdes, les huiles essentielles, ou de façon générale des composés odoriférants naturels ou synthétiques,
- environ 10% de solvo-surfactif,
- environ 0,1% à 2% de tensioactif et notamment de 0,5%, 1% ou 2%, ou
   environ 0,2% à 2% de tensioactif.
les microémulsions de la présente description sont constituées
- d'environ 80% d'eau,
- d'environ 10% d'une substance parfumante choisie parmi un terpène, un terpénoïde, une huile essentielle, ou de façon générale un composé naturel ou synthétique présentant des propriétés odoriférantes, notamment choisi parmi les aldéhydes, les esters, les éthers, les cétones, les ionones, ladite substance parfumante n'étant pas un mélange,
- d'environ 10% de solvo-surfactif ,
- et d'environ 0,1% à 2% de tensioactif et notamment de 0,5%, 1% ou 2%.

De façon particulière, les microémulsions de la présente description sont constituées
- d'environ 80% d'eau,
- d'environ 10% d'une substance parfumante choisie parmi une huile essentielle,
- d'environ 10% de solvo-surfactif C₆E₄,
- et d'environ 0,1% à 2% de tensioactif et notamment de 0,5%, 1% ou 2%.
De façon avantageuse, les microémulsions de la présente description sont constituées
- d'environ 80% d'eau,
- d'environ 10% d'une substance parfumante choisie parmi une huile essentielle,
- d'environ 10% de solvo-surfactif ,
- et d'environ 0,1% à 2% de tensioactif anionique et notamment de 0,5%, 1% ou 2%, de tensioactif anionique, de préférence de type alkylsulfate ou propoxysulfate, en particulier propoxysulfate.

Les microémulsions de l'invention sont constituées de :
- 87,67% d'eau, 4,75% de composition parfumante P, 6,64% de C₆E₄, 0,94% de Alf8S,
- 86,04% d'eau, 5,00% de composition parfumante P, 8,01% de C₆E₄, 0,95% de Alf4S,
- 87,69% d'eau, 4,77% de composition parfumante P, 6,45% de C₆E₄, 1,09% de DHS,
- 87,41% d'eau, 4,82% de composition parfumante P, 6,65% de C₆E₄, 1,12% de [DiC₁₀][Cl],
- 88,86% d'eau, 4,81% de composition parfumante PF1, 5,39% de C₆E₄, 0,94% de Alf8S,
- 86,08% d'eau, 4,89% de composition parfumante PF1, 8,00% de C₆E₄, 1,03% de Alf4S,
- 87,75% d'eau, 4,87% de composition parfumante PF1, 6,16% de C₆E₄, 1,22% de DHS,
- 88,87% d'eau, 4,90% de composition parfumante PF1, 5,18% de C₆E₄, 1,05% de [DiC₁₀][Cl],
- 82,49% d'eau, 4,49% de composition parfumante PF1, 12,1% de C₆E₄, 0,92% de CAPB,
- 87,36% d'eau, 4,79% de composition parfumante PF2, 6,95% de C₆E₄, 0,90% de Alf4S,
- 86,66% d'eau, 4,90% de composition parfumante PF2, 7,30% de C₆E₄, 1,14% de DHS,
- 86,54% d'eau, 5,25% de composition parfumante PF2, 7,11% de C₆E₄, 1,10% de [DiC₁₀][Cl],
- 87,71% d'eau, 4,84% de composition parfumante PF3, 6,29% de C₆E₄, 1,16% de [DiC₁₀][Cl],
- 84,81% d'eau, 4,88% de composition parfumante PF3, 9,21% de C₆E₄, 1,10% de DHS,
- 86,25% d'eau, 4,88% de composition parfumante PF3, 7,90% de C₆E₄, 0,97% de Alf4S,
- 86,91% d'eau, 4,85% de composition parfumante PF3, 7,28% de C₆E₄, 0,96% de Alf8S, dans lesquelles
   - P est une composition parfumante complexe,
   - PF1 est une composition parfumante contenant, en pourcentages massiques, 1,4% d'a-pinène, 1,5% de β-pinène, 0,4% de camphène, 21,3% de cinéole, 2,7% d'ocimène, 7,7% de camphre, 19,2% de dipropylène glycol, 36,7% de linalol, 7,4% d'acétate de linalyle et 1,7% de limonène,
   - PF2 est une composition parfumante contenant, en pourcentages massiques, 1,4% d'a-pinène, 1,5% de β-pinène, 0,4% de camphène, 21,3% de cinéole, 2,7% d'ocimène, 7,7% de camphre, 19,2% de diéthylphtalate, 36,7% de linalol, 7,4% d'acétate de linalyle et 1,7% de limonène,
   - PF3 est une composition parfumante contenant, en pourcentages massiques, 1,4% d'a-pinène, 1,5% de β-pinène, 0,4% de camphène, 21,3% de cinéole, 2,7% d'ocimène, 7,7% de camphre, 19,2% de myristate d'isopropyle, 36,7% de linalol, 7,4% d'acétate de linalyle et 1,7% de limonène.

Les microémulsions de l'invention sont constituées de :
- 80,50% d'eau, 10,38% de composition parfumante PF1, 7,48% de C₆E₄, 1,64% de [DiC₁₀][Cl],
- 81,40% d'eau, 10,66% de composition parfumante PF1, 5,72% de C₆E₄, 2,22% de [DiC₁₀][Cl],
- 85,69% d'eau, 3,62% de composition parfumante PF1, 9,74% de C₆E₂, 0,95% de Alf4S,
- 85,43% d'eau, 3,59% de composition parfumante PF1, 9,56% de C₆E₂, 1,42% de Alf8S,
- 85,29% d'eau, 4,74% de composition parfumante PF1, 9,50% de C₆E₃, 0,47% de Alf4S,
- 85,32% d'eau, 4,73% de composition parfumante PF1, 9,44% de C₆E₃, 0,51% de Alf8S,
- 85,32% d'eau, 4,71% de composition parfumante PF1, 9,80% de C₆E₃, 0,17% de DHS,
- 85,42% d'eau, 4,73% de composition parfumante PF1, 9,37% de C₆E₃, 0,48% de Alf8S,
- 85,26% d'eau, 4,74% de composition parfumante PF1, 9,54% de C₆E₃, 0,46% de CTaBr,
- 85,02% d'eau, 4,74% de composition parfumante PF1, 9,75% de C₆E₃, 0,49% de SDBS,
- 85,12% d'eau, 4,71% de composition parfumante PF1, 9,69% de C₆E₃, 0,48% de [DiC₁₀][Cl],
- 85,12% d'eau, 4,71% de composition parfumante PF1, 9,69% de C₄Gly/C₆E₂ 1/1, 0,48% de SDS,
   dans lesquelles
   - PF1 est une composition parfumante contenant, en pourcentages massiques, 1,4% d'a-pinène, 1,5% de β-pinène, 0,4% de camphène, 21,3% de cinéole, 2,7% d'ocimène, 7,7% de camphre, 19,2% de dipropylène glycol, 36,7% de linalol, 7,4% d'acétate de linalyle et 1,7% de limonène.

Les microémulsions de l'invention sont avantageusement utilisées pour la préparation de compositions appliquées à :
- la parfumerie fine,
- la détergence, le textile et le nettoyage des surfaces dures,
- la cosmétique et les produits d'hygiène corporelle,
- la désinfection,
- le phytosanitaire,
- la pharmacie.

Des compositions peuvent être aussi utilisées en cosmétique; les microémulsions peuvent alors contenir un ou plusieurs des composés choisis parmi : des silicones, de l'huile paraffine, de l'isooctane, de l'isodécane, du squalène, du squalane, du sébum, de la lanoline.

Des compositions peuvent être utilisées aussi pour le nettoyage des surfaces dures; les microémulsions peuvent alors contenir un ou plusieurs des composés choisis parmi des huiles végétales en particulier des trioléines, des coupes pétrolières lourdes, de l'huile paraffine épaisse, des savons d'aluminium ou de calcium.

La présente description concerne également des microémulsions de type huile/eau consistant en
- 80% à 94% d'eau,
- 1% à 15% de substances hydrophobes, en particulier 3% à 10%, notamment environ 10%,
- 15% à 65%, notamment 20% à 60%, notamment 15% à 30%, d'un ou de plusieurs solvo-surfactifs volatils,
- 0% de tensioactif,
le solvo-surfactif ou le mélange de solvo-surfactifs ne contenant pas plus de 2% de composés non-volatils,
lequel solvo-surfactif est un composé amphiphile choisi parmi :
▪ les alcools gras polyéthoxylés CᵢEⱼ à chaîne carbonée linéaire, de formule CH₃-(CH₂)ᵢ₋₁-(O-CH₂-CH₂)ⱼ-OH,
   ou les alcools gras polyéthoxylés à chaîne ramifiée, de formule CᵢH₂ᵢ₊₁(OCH₂CH₂)ⱼOH,
   dans lesquelles
   - i représente le nombre d'atomes de carbone de la chaîne hydrocarbonée linéaire ou ramifiée et varie de 3 à 8, notamment varie de 4 à 6,
   - j représente le nombre d'oxydes d'éthylène et varie de 1 à 4, notamment varie de 2 à 4,
▪ les dérivés de glycérol CₖGly de formule 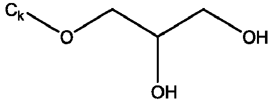 dans laquelle Cₖ représente
   - un groupe alkyle, linéaire ou ramifié, contenant de 1 à 6 atomes de carbone, en particulier de 3 à 5 atomes de carbone, notamment 4 ou 5 atomes de carbone, notamment C₄Gly de formule 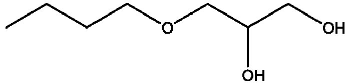 ou C₅Gly de formule 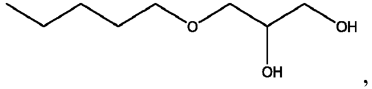 ou notamment le dérivé ramifié i-C₅Gly de formule 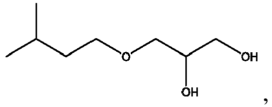
   - un groupe acyle, linéaire ou ramifié, contenant de 2 à 6 atomes de carbone, en particulier de 3 à 5 atomes de carbone, notamment 4 ou 5 atomes de carbone, notamment le pentanoate de glycéryle de formule 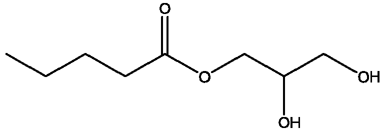
▪ les dérivés d'isosorbide, IsosorbideCₘ₁ et IsosorbideCₘ₂, les dérivés d'isomannide, IsomannideCₘ₃, les dérivés d'isoidide, IsoidideCₘ₄ de formules 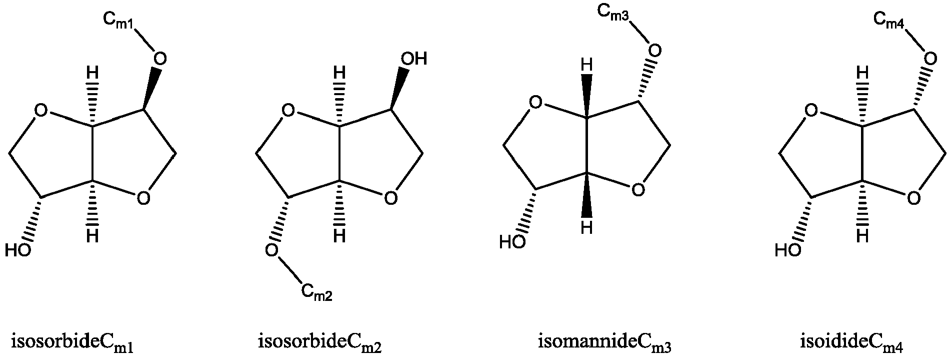 dans lesquelles Cₘ₁, Cₘ₂, Cₘ₃ et Cₘ₄ représentent
   - un groupe alkyle, linéaire ou ramifié, contenant de 1 à 6 atomes de carbone, en particulier de 3 à 5 atomes de carbone, notamment 4 ou 5 atomes de carbone,
   - un groupe acyle, linéaire ou ramifié, contenant de 2 à 6 atomes de carbone, en particulier de 3 à 5 atomes de carbone, notamment 4 ou 5 atomes de carbone.
   Cet aspect est illustré par l'exemple 11, numéros 11-A, 11-B, 11-C et 11-D.

La FIGURE 1 représente la température pour laquelle 98% en masse de l'échantillon étudié par analyse thermogravimétrique (ATG_{98%}) sont évaporés, à pression atmosphérique, en fonction du nombre d'atomes de carbone du composé. Les trois catégories de composés "COV très volatils", "COV volatils" et "COV semi-volatils", sont définies ci-dessus.
La **FIGURE 2** représente les photographies des microémulsions de l'exemple 7, numéros 9-3, 9-4, 9-5, 9-6, 9-7, 9-8, 9-9, 9-10.
Les **FIGURES 3 à 6** représentent les photographies des microémulsions de l'exemple 7, numéros 9-5, 9-7, 9-8, et 9-9, ainsi que les photographies des tissus sur lesquels a été déposée la microémulsion correspondante. Le dépôt a été réalisé au moyen d'une pipette en contenant 300µL. En quelques heures (5 heures) à température ambiante (20-22°C°C), ils ne sont plus visibles sur le tissu et aucune trace n'apparaît.

### EXEMPLES

### Huiles essentielles étudiées (fournies par la Copper):

- Ylang Ylang (**Yl-Yl**)
- bergamote **(Berg)**
- Eucalyptus **(Eucal)**
- Lavande **(Lav)**
- Menthe poivrée **(Menth)**
- Lemon Grass **(Lem)**
- Huile de pin **(Pin)**
- Huile de patchouli **(Patch)**

### Solvo-surfactifs étudiés :

- **C₆E₂** (Aldrich)
- **C₆E₃** synthétisé : éther de triéthylène glycol monohexyl
   comportant une chaîne hydrocarbonée à 6 atomes de carbone et 3 motifs oxydes d'éthylène,
- **C₆E₄** synthétisé : éther de tétraéthylène glycol monohexyl comportant une chaîne hydrocarbonée à 6 atomes de carbone et 4 motifs oxydes d'éthylène,
- **C₄Gly** synthétisé,
- **C₅Gly** synthétisé.

### Tensioactifs étudiés en tant que « boosters » :

- **CAPB** Cocoamidopropylbétaïne (fournisseur Cognis)
- **[DiC₁₀][Cl]** Chlorure de didécyldiméthyl ammonium (fournisseur Aldrich)
- **DHS** Dihexylsulfosuccinate (fournisseur Aldrich)
- **Alf4S** Alfoterra 4S (fournisseur Sasol)
- **Alf8S** Alfoterra 8S (fournisseur Sasol)

Les composés Alfoterra sont des tensioactifs anioniques contenant des sulfates de propoxy monoalkyles branchés comprenant de 6 à 26 atomes de carbones
- **SDS** dodécylsulfate de sodium (Aldrich)
- **CTaBr** Bromure de cétyltriméthylammonium,
- **SDBS** sodium dodécylbenzène sulfonate de sodium.

**Caractéristiques/types des appareils d'analyse utilisés pour l'analyse thermogravimétrique :**
appareil TGA Q5000 de chez TA Instruments,
débits : tête de balance : 10 mL / min, four : 25 mL / min,
vitesse de chauffe maxi 500°C / min, Tmax 1200°C,

Portée de la balance 5 g, perte mesurable 100 mg, masse recommandée 5 mg, nacelle platine HT.

### Substances parfumantes testées : formule générale

Les formules parfumantes utilisées dans les compositions du brevet US1994/5374614 (ou dans exemple 2 de EP0572080) ont été réalisées et testées avec les systèmes de l'invention. La formule de base figure dans le tableau 1 ci-après, les différents composants étant renseignés au moyen de leur pourcentage massique. Le solvant a été introduit avec un pourcentage massique de 19,2 %.

**Formule de base dune composition parfumante**

| Molécule | % massique |
|---|---|
| α-Pinène | 1,4 |
| β-Pinène | 1,5 |
| Camphène | 0,4 |
| Cinéole | 21,3 |
| Ocimène | 2,7 |
| Camphre | 7,7 |
| **Solvant** | **19,2** |
| Linalol | 36,7 |
| Acétate de linalyle | 7,4 |
| Limonène | 1,7 |

### Substances parfumantes testées : formules particulières

Elles sont réalisées à partir de la formule générale définie ci-dessus et sont désignées par PF1, PF2 et PF3. Les pourcentages massiques de leurs composants sont indiqués dans les tableaux ci-après.
- Le solvant de PF1 est le dipropylène glycol,
- le solvant de PF2 est le phtalate de diéthyle,
- Le solvant de PF3 est le myristate d'isopropyle.

**Composition PF1**

| Molécule | % massique |
|---|---|
| α-Pinène | 1,4 |
| β-Pinène | 1,5 |
| Camphène | 0,4 |
| Cinéole | 21,3 |
| Ocimène | 2,7 |
| Camphre | 7,7 |
| Dipropylène glycol | 19,2 |
| Linalol | 36,7 |
| Acétate de linalyle | 7,4 |
| Limonène | 1,7 |

**Composition PF2**

| Molécule | % massique |
|---|---|
| α-Pinène | 1,4 |
| β-Pinène | 1,5 |
| Camphène | 0,4 |
| Cinéole | 21,3 |
| Ocimene | 2,7 |
| Camphre | 7,7 |
| **Phtalate de diéthyle** | **19,2** |
| Linalol | 36,7 |
| Linalyl acetate | 7,4 |
| Limonène | 1,7 |

**Composition PF3**

| Molécule | % massique |
|---|---|
| α-Pinène | 1,4 |
| β-Pinène | 1,5 |
| Camphène | 0,4 |
| Cinéole | 21,3 |
| Ocimène | 2,7 |
| Camphre | 7,7 |
| **Myristate d' isopropyle** | **19,2** |
| Linalol | 36,7 |
| Acétate de linalyle | 7,4 |
| Limonène | 1,7 |

### ESSAIS COMPARATIFS

### Composition réalisée selon l'art antérieur

- 5% de parfum PF1
- 85% eau
- 9% nonyl EO(9) phényl éther
- 1% Sodium dodécyl sulfate

### Composition selon l'invention :

- 5% de parfum PF1
- 85% eau
   - 9% C6E4
   - 1% Sodium dodécyl sulfate

La composition selon l'invention est limpide à 5°C, 20°C, et à 50 °C ; celle réalisée selon l'art antérieur est trouble à toutes les températures testées.

### Exemple 1 : Systèmes pour la solubilisation d'environ 5 % de la substance parfumante avec le solvo-surfactif C₆E₄

Dans le tableau 1 figurant ci-après, PF1, PF2 et PF3 sont les compositions parfumantes précédemment désignées. Par "P", on désigne une composition parfumante utilisée en parfumerie fine, sa formulation n'étant pas connue ou confidentielle.

Par **"booster",** on désigne le tensioactif.

**Les pourcentages sont des pourcentages massiques**

**Tableau 1**

| **Système** | **Parfum** | **% Parfum** | **%Eau** | **% Booster** | **% C₆E₄** |
|---|---|---|---|---|---|
| C₆E₄/Alf8S | P | 4,75 | 87,67 | 0,94 | 6,64 |
| C₆E₄/Alf4S | P | 5,00 | 86,04 | 0,95 | 8,01 |
| C₆E₄/DHS | P | 4,77 | 87,69 | 1,09 | 6,45 |
| C₆E₄/[DiC₁₀][Cl] | P | 4,82 | 87,41 | 1,12 | 6,65 |
| C₆E₄/Alf8S | PF1 | 4,81 | 88,86 | 0,94 | 5,39 |
| C₆E₄/Alf4S | PF1 | 4,89 | 86,08 | 1,03 | 8,00 |
| C₆E₄/DHS | PF1 | 4,87 | 87,75 | 1,22 | 6,16 |
| C₆E₄/[DiC₁₀][Cl] | PF1 | 4,90 | 88,87 | 1,05 | 5,18 |
| C₆E₄/CAPB | PF1 | 4,49 | 82,49 | 0,92 | 12,10 |
| C₆E₄/Alf4S | PF2 | 4,79 | 87,36 | 0,90 | 6,95 |
| C₆E₄/DHS | PF2 | 4,90 | 86,66 | 1,14 | 7,30 |
| C₆E₄/C₁₀C₁₀Cl | PF2 | 5,25 | 86,54 | 1,10 | 7,11 |
| C₆E₄/C₁₀C₁₀Cl | PF3 | 4,84 | 87,71 | 1,16 | 6,29 |
| C₆E₄/DHS | PF3 | 4,88 | 84,85 | 1,06 | 9,21 |
| C₆E₄/Alf4S | PF3 | 4,88 | 86,25 | 0,97 | 7,90 |
| C₆E₄/Alf8S | PF3 | 4,85 | 86,91 | 0,96 | 7,28 |

### Exemple 2 : systèmes pour la solubilisation d'huiles essentielles en présence de 10% de C₆E₄

Les compositions parfumantes de ces systèmes contiennent environ 10 % du solvo-surfactif **C₆E₄**, environ 10 % d'huiles essentielles, environ 80 % d'eau. Les pourcentages de tensioactif "booster" sont égaux à 0,5 %, ou à 1 %, ou à 2%.
Les pourcentages indiqués sont des pourcentages massiques.

Les différents pourcentages de composants de ces systèmes figurent dans le tableau 2 représenté ci-après :

**Tableau 2**

| **Huile** | **Booster** | **% Booster** |
|---|---|---|
| Y1-Y1 | Alf4S | 2 |
| Berg | DHS | 2 |
| Berg | Alf8S | 1 |
| Eucal | [DiC₁₀][Cl] | 0,5 |
| Eucal | [DiC₁₀][Cl] | 1 |
| Eucal | DHS | 2 |
| Eucal | Alf4S | 0,5 |
| Eucal | Alf8S | 1 |
| Lav | [DiC₁₀][Cl] | 0,5 |
| Lav | Alf4S | 1 |
| Menth | [DiC₁₀][Cl] | 1 |
| Menth | Alf8S | 2 |
| Lem | [DiC₁₀][Cl] | 0,5 |
| Lem | Alf8S | 1 |

### Exemple 3 : systèmes contenant le composé C₆E₂ en tant que solvo-surfactif, pour la solubilisation d'environ 3% à 10% de la composition parfumante PF1

Dans le tableau 3 figurant ci-après, PF1 est la composition parfumante précédemment désignée. Par **"booster",** on désigne le tensioactif. Les pourcentages indiqués sont des pourcentages massiques.

**Tableau 3**

| **Booster Nature et pourcentage** | **eau** | **C₆E₂** | **PF1** |
|---|---|---|---|
| **Alf4S 0,95** | **85,69** | **9,74** | **3,62** |
| Alf4S 1,43 | 85,09 | 9,89 | 3,59 |
| Alf4S 1,89 | 85,10 | 9,41 | 3,60 |
| **Alf8S 1,42** | **85,43** | **9,56** | **3,59** |
| Alf8S 1,89 | 85,10 | 9,41 | 3,60 |
| Alf8S 1,82 | 82,15 | 9,08 | 6,95 |
| **DHS 1,80** | **82,20** | **9,07** | **6,93** |
| **SDS 1,39** | **81,93** | **9,81** | **6,87** |
| SDS 1,81 | 82,22 | 9,04 | 6,93 |

### Exemple 4 : systèmes contenant le composé C₆E₃ en tant que solvo-surfactif, pour la solubilisation d'environ 3% à 10% de la composition parfumante

Dans le tableau 4 figurant ci-après, PF1 est la composition parfumante précédemment désignée. Les pourcentages indiqués sont des pourcentages massiques.

**Tableau 4**

| **Booster Nature et pourcentage** | | **eau** | **C₆E₃** | **PF1** |
|---|---|---|---|---|
| Alf4S | 0,47 | 85,29 | 9,50 | 4,74 |
| Alf4S | 0,95 | 84,97 | 9,37 | 4,71 |
| Alf4S | 1,41 | 84,50 | 9,40 | 4,69 |
| Alf4S | 1,87 | 83,90 | 9,57 | 4,66 |
| Alf4S | 1,34 | 80,73 | 8,98 | 8,95 |
| Alf4S | 1,78 | 80,18 | 9,14 | 8,90 |
| Alf8S | 0,51 | 85,32 | 9,44 | 4,73 |
| Alf8S | 1,00 | 84,86 | 9,42 | 4,72 |
| Alf8S | 1,47 | 84,20 | 9,67 | 4,66 |
| Alf8S | 1,93 | 83,79 | 9,64 | 4,64 |
| Alf8S | 1,41 | 80,44 | 9,24 | 8,91 |
| Alf8S | 1,84 | 80,08 | 9,21 | 8,87 |
| DHS | 0,34 | 85,29 | 9,66 | 4,71 |
| DHS | 0,50 | 85,29 | 9,51 | 4,70 |
| DHS | 0,68 | 85,34 | 9,31 | 4,67 |
| DHS | 1,65 | 80,53 | 8,89 | 8,93 |
| SDS | 0,48 | 85,42 | 9,37 | 4,73 |
| SDS | 0,97 | 85,08 | 9,29 | 4,68 |
| SDS | 1,38 | 84,42 | 9,54 | 4,66 |
| SDS | 1,87 | 84,16 | 9,31 | 4,66 |
| SDS | 1,79 | 80,41 | 8,89 | 8,91 |
| CTaBr | 0,46 | 85,26 | 9,54 | 4,74 |
| CTaBr | 0,98 | 84,74 | 9,59 | 4,69 |
| CTaBr | 1,37 | 84,36 | 9,57 | 4,70 |
| CTaBr | 1,91 | 84,23 | 9,21 | 4,65 |
| SDBS | 0,49 | 85,02 | 9,75 | 4,74 |
| SDBS | 0,92 | 84,78 | 9,57 | 4,73 |
| SDBS | 1,39 | 84,61 | 9,31 | 4,69 |
| SDBS | 1,86 | 84,03 | 9,45 | 4,66 |
| [DiC₁₀][Cl] | 0,48 | 85,12 | 9,69 | 4,71 |
| [DiC₁₀][Cl] | 0,94 | 84,90 | 9,45 | 4,71 |
| [DiC₁₀][Cl] | 1,38 | 84,53 | 9,39 | 4,70 |
| [DiC₁₀][Cl] | 1,92 | 83,80 | 9,62 | 4,66 |
| [DiC₁₀][Cl] | 1,32 | 80,73 | 8,97 | 8,98 |
| [DiC₁₀][Cl] | 1,83 | 80,07 | 9,19 | 8,91 |

### Exemple 5 : systèmes contenant le composé C₅Gly en tant que solvo-surfactif, pour la solubilisation d'environ 3% à 10% de la composition parfumante

Ces systèmes ne tombent pas dans l'étendue de protection revendiquée.

Dans le tableau 5 figurant ci-après, PF1 est la composition parfumante précédemment désignée. Les pourcentages indiqués sont des pourcentages massiques.

**Tableau 5**

| **Booster Nature et pourcentage** | | **eau** | **C₅Gly** | **PF1** |
|---|---|---|---|---|
| Alf4S | 0,47 | 85,29 | 9,50 | 4,74 |
| Alf4S | 0,95 | 84,97 | 9,37 | 4,71 |
| Alf4S | 1,41 | 84,50 | 9,40 | 4,69 |
| Alf4S | 1,87 | 83,90 | 9,57 | 4,66 |
| Alf4S | 1,34 | 80,73 | 8,98 | 8,95 |
| Alf4S | 1,78 | 80,18 | 9,14 | 8,90 |
| Alf8S | 0,51 | 85,32 | 9,44 | 4,73 |
| Alf8S | 1,00 | 84,86 | 9,42 | 4,72 |
| Alf8S | 1,47 | 84,20 | 9,67 | 4,66 |
| Alf8S | 1,93 | 83,79 | 9,64 | 4,64 |
| Alf8S | 1,41 | 80,44 | 9,24 | 8,91 |
| Alf8S | 1,84 | 80,08 | 9,21 | 8,87 |
| DHS | 0,34 | 85,29 | 9,66 | 4,71 |
| DHS | 0,50 | 85,29 | 9,51 | 4,70 |
| DHS | 0,68 | 85,34 | 9,31 | 4,67 |
| DHS | 1,65 | 80,53 | 8,89 | 8,93 |
| SDS | 0,48 | 85,42 | 9,37 | 4,73 |
| SDS | 0,97 | 85,06 | 9,29 | 4,68 |
| SDS | 1,38 | 84,42 | 9,54 | 4,66 |
| SDS | 1,87 | 84,16 | 9,31 | 4,66 |
| SDS | 1,79 | 80,41 | 8,89 | 8,91 |
| CTaBr | 0,46 | 85,26 | 9,54 | 4,74 |
| CTaBr | 0,98 | 84,74 | 9,59 | 4,69 |
| CTaBr | 1,37 | 84,36 | 9,57 | 4,70 |
| CTaBr | 1,91 | 84,23 | 9,21 | 4,65 |
| SDBS | 0,49 | 85,02 | 9,75 | 4,74 |
| SDBS | 0,92 | 84,78 | 9,57 | 4,73 |
| SDBS | 1,39 | 84,61 | 9,31 | 4,69 |
| SDBS | 1,86 | 84,03 | 9,45 | 4,66 |
| [DiC₁₀][Cl] | 0,48 | 85,12 | 9,69 | 4,71 |
| [DiC₁₀][Cl] | 0,94 | 84,90 | 9,45 | 4,71 |
| [DiC₁₀][Cl] | 1,38 | 84,53 | 9,39 | 4,70 |
| [DiC₁₀][Cl] | 1,92 | 83,80 | 9,62 | 4,66 |
| [DiC₁₀][Cl] | 1,32 | 80,73 | 8,97 | 8,98 |
| [DiC₁₀][Cl] | 1,83 | 80,07 | 9,19 | 8,91 |

### Exemple 6 : système contenant le mélange de solvo-surfactifs C₄Gly/C₆E₂ dans la proportion 1/1 pour la solubilisation d'environ 5% de la composition parfumante

Dans le tableau 6 figurant ci-après, PF1 est la composition parfumante précédemment désignée. Les pourcentages indiqués sont des pourcentages massiques.

**Tableau 6**

| **Booster Nature et pourcentage** | | **eau** | **C₄Gly + C₆E₂** | **PF1** |
|---|---|---|---|---|
| **SDS** | **0,48** | **85,12** | **9,69** | **4,71** |

### Exemple 7 : systèmes contenant un mélange de solvo-surfactif et de tensioactif "booster" pour la solubilisation de 3% à 10% de composition parfumante PF1, avec photo des systèmes

Dans le tableau 7 figurant ci-après, PF1 est la composition parfumante précédemment désignée. Les pourcentages indiqués sont des pourcentages massiques.
Dans le cas des boosters, ces pourcentages ont été corrigés à partir du % de la solution aqueuse commerciale utilisée (DHS à 80% dans l'eau - Alf 4S à 28% - Alf 8S à 27% - SDS pur).

**Tableau 7**

| **N°** | **Composition de l'invention** | | | | **Photo** |
|---|---|---|---|---|---|
| | **Solvo-surfactif** | **Booster** | **Eau** | **PF1** | |
| **9-3** | C6E4 | SDS | 86,1 | 3,7 | Cf figure 2 |
| | 9,7 | 0,5 | | | |
| **9-4** | C6E4 | SDS | 82,8 | 7,1 | Cf figure 2 |
| | 9,6 | 0,5 | | | |
| **9-5** | C6E3 | Alf4S | 84,7 | 4,9 | Cf figure 2, figure 3 |
| | 9,4 | 1,0 | | | |
| **9-6** | C6E3 | DHS | 84,6 | 4,7 | Cf figure 2 |
| | 9,7 | 1,0 | | | |
| **9-7** | C6E3 | Alf8S | 80,0 | 9,0 | Cf figure 2, Figure 4 |
| | 9,2 | 1,8 | | | |
| **9-8** | C5Gly | Alf4S | 85,95 | 4,8 | Cf figure 2, Figure 5 |
| | 8,3 | 0,95 | | | |
| **9-9** | C5Gly | Alf4S | 81,35 | 8,2 | Cf figure 2, Figure 6 |
| | 9 | 1,45 | | | |
| **9-10** | C5Gly | DHS | 84,8 | 4,7 | Cf figure 2 |
| | 9,5 | 1,0 | | | |

Les microémulsions selon l'invention sont limpides et stable (Figure 2).

Les compositions 9-8, 9-9 and 9-10 ne tombent pas dans l'étendue de protection revendiquée.

### Exemple 8 : protocole de préparation des microémulsions

Les microémulsions, systèmes thermodynamiquement stables, sont obtenues instantanément dès que les différents constituants sont mélangés sans apport d'énergie particulier. Ainsi, le solvo-surfactif, l'eau, la substance parfumante et le booster sont pesés dans le même flacon, lequel est ensuite légèrement secoué pour homogénéiser le mélange. L'ordre d'introduction des constituants du mélange n'a pas d'importance. La microémulsion transparente et monophasique à l'échelle macroscopique se forme instantanément.

## Revendications

1. Microémulsions de type huile/eau consistant en, en pourcentage massique :
• au moins 80% d'eau,
• 1% à 10% de substances hydrophobes, en particulier 3% à 10%, notamment environ 10%,
• 5% à 15%, notamment 5% à 10%, d'un ou de plusieurs solvo-surfactifs dont la température d'ébullition est inférieure à 250 °C à pression atmosphérique,
• 0,1% à 2% d'un ou plusieurs tensioactifs, en pourcentage massique,
la composition contenant la substance hydrophobe, l'eau, le solvo-surfactif et le tensioactif contenant au plus 2 % par rapport à la masse totale de la composition de composés dont la température d'ébullition est supérieure à 250 °C à pression atmosphérique,
un des solvo-surfactifs étant un composé amphiphile choisi parmi :
▪ les alcools gras polyéthoxylés CᵢEⱼ à chaîne carbonée linéaire, de formule CH₃-(CH₂)ᵢ₋₁-(O-CH₂-CH₂)ⱼ-OH,
ou les alcools gras polyéthoxylés à chaîne ramifiée, de formule CᵢH₂ᵢ₊₁(OCH₂CH₂)ⱼOH,
dans lesquelles
• i représente le nombre d'atomes de carbone de la chaîne hydrocarbonée linéaire ou ramifiée et varie de 4 à 6,
• j représente le nombre d'oxydes d'éthylène et varie de 2 à 4,
lequel tensioactif est choisi parmi
• les tensioactifs anioniques,
• les tensioactifs cationiques,
• les tensioactifs amphotères, et
• les tensioactifs non-ioniques.

2. Microémulsions de type huile/eau consistant en, en pourcentage massique :
• au moins 80% d'eau,
• 1% à 10% de substances hydrophobes, en particulier 3% à 10%, notamment environ 10%,
• 5% à 15%, notamment 5% à 10%, d'un ou de plusieurs solvo-surfactifs dont la température d'ébullition est inférieure à 250 °C à pression atmosphérique,
• 0,1% à 2% d'un ou plusieurs tensioactifs, en pourcentage massique,
la composition contenant la substance hydrophobe, l'eau, le solvo-surfactif et le tensioactif contenant au plus 2 % par rapport à la masse totale de la composition de composés dont la température d'ébullition est supérieure à 250 °C à pression atmosphérique,
un des solvo-surfactifs étant un composé amphiphile choisi parmi :
C₄E₁, C₅E₁, C₅E₂, C₅E₃, C₆E₂, C₆E₃, C₆E₄,
lequel tensioactif est choisi parmi
• les tensioactifs anioniques,
• les tensioactifs cationiques,
• les tensioactifs amphotères, et
• les tensioactifs non-ioniques.

3. Microémulsions selon l'une des revendications 1 ou 2, dans lesquelles la substance hydrophobe est une substance parfumante choisie parmi un terpène, un terpénoïde, une huile essentielle, ou un composé synthétique présentant des propriétés odoriférantes, notamment choisi parmi les aldéhydes, les esters, les cétones, les ionones, les éthers,
ladite substance parfumante pouvant être une composition parfumante complexe, contenant un mélange de plusieurs terpènes, terpénoïdes, huiles essentielles, ou plusieurs composés odoriférants synthétiques purs,
le pourcentage massique de ladite substance parfumante étant compris de 1% à 10%, et notamment de 3% à 10%, et étant en particulier environ égal à 5% ou environ égal à 10%.

4. Microémulsions selon l'une des revendications 1 à 3, dans lesquelles les solvo-surfactifs sont des composés amphiphiles choisis parmi C₅E₂, C₆E₂, C₆E₃ ou C₆E₄.

5. Microémulsions selon l'une des revendications 1 à 4, dans lesquelles les solvo-surfactifs sont utilisés en mélange d'au moins deux composés solvo-surfactifs,
le mélange étant en particulier constitué d'au moins 2 deux CᵢEⱼ,
ou d'un CᵢEⱼ et au moins un dérivé de glycérol CₖGly de formule : 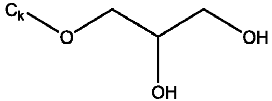 dans laquelle Cₖ représente
• un groupe alkyle, linéaire ou ramifié, contenant de 1 à 6 atomes de carbone, en particulier de 3 à 5 atomes de carbone, notamment 4 ou 5 atomes de carbone, notamment C₄Gly de formule 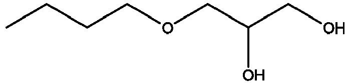 ou C₅Gly de formule 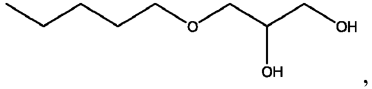 ou notamment le dérivé ramifié i-C₅Gly de formule 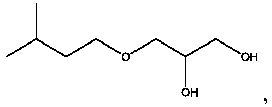
• un groupe acyle, linéaire ou ramifié, contenant de 2 à 6 atomes de carbone, en particulier de 3 à 5 atomes de carbone, notamment 4 ou 5 atomes de carbone, notamment le pentanoate de glycéryle de formule 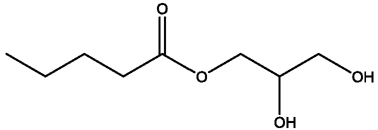 ou d'un CᵢEⱼ et au moins un dérivé d'isosorbide, d'isomannide, ou d'isoidide,
le mélange étant notamment constitué de C₆E₂ et de C₄Gly, dans les proportions de 1 pour 1 en volume.

6. Microémulsions selon l'une des revendications 1 à 5, dans lesquelles les tensioactifs sont anioniques et représentent
• les alkylsulfonates, notamment le sulfosuccinate de dihexyle (DHS) de formule 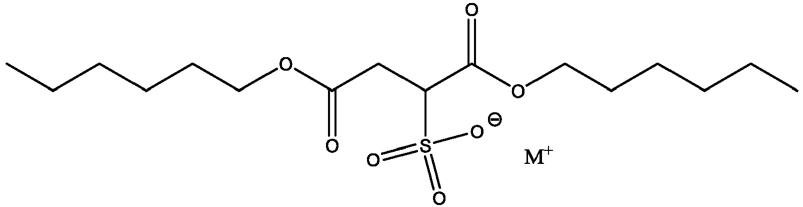 dans laquelle
M⁺ représente Na⁺, K⁺, NH₄⁺, (HOCH₂CH₂)₃NH⁺,
ou notamment le sulfosuccinate d'éthyl-2-hexyle de formule 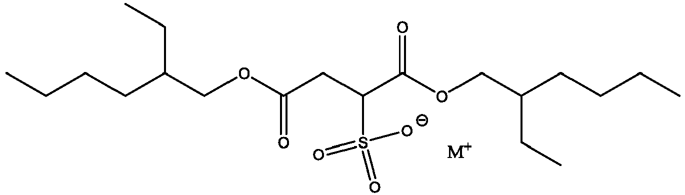 dans laquelle
M⁺ représente Na⁺, K⁺, NH₄⁺, (HOCH₂CH₂)₃NH⁺,
• les alkylarylsulfonates de formule 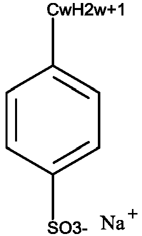 dans laquelle w est un entier compris de 8 à 12,
en particulier l'isooctyl, l'isononyl et notamment l'isododécylbenzènesulfonate de sodium SDBS de formule 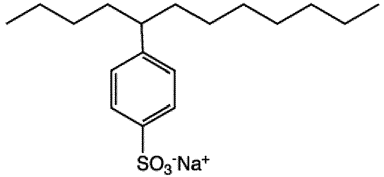
• les propoxysulfates, notamment les composés de formule 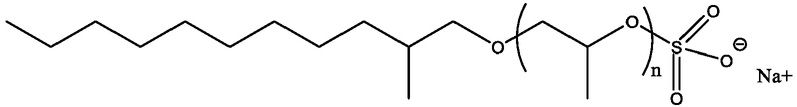 dans laquelle le nombre de motifs propoxylate n est compris de 4 à 8,
□ n pouvant être notamment égal à 4, (Alf4S)
□ n pouvant être notamment égal à 8, (Alf8S)
• ou les alkylsulfates, notamment les sels du sulfate de lauryle tel que le dodécyl sulfate de sodium (SDS) et les alkyléther sulfates de sodium tel que le lauryléther sulfate de sodium (LESNa),
• ou les sels d'acides gras de formule R-CO₂⁻M⁺,
dans laquelle R représente une chaîne carbonée, linéaire ou ramifiée, saturée ou insaturée, contenant 8 à 18 atomes de carbone, et M⁺ représente un cation choisi parmi les ions Na⁺, K⁺, NH₄⁺, (HOCH₂CH₂)₃NH⁺,
notamment le sel d'acide oléique de formule CH₃(CH₂)₇CH=CH(CH₂)₇CO₂⁻M⁺ dans lequel M⁺ a les significations définies ci-dessus,
ou dans lesquelles les tensioactifs sont cationiques et sont des sels d'ammonium choisis parmi
• des sels de tétraalkylammonium, notamment des sels de dialkyldiméthylammonium de formule 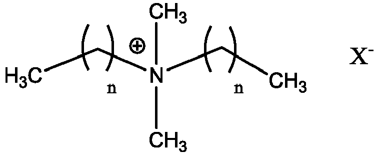 dans laquelle
X⁻ représente un anion choisi parmi Cl⁻, Br⁻, I⁻, CH₃COO⁻ ou lactate,
n est le nombre de méthylène compris de 6 à 12,
et représente notamment un sel de didécyl diméthylammonium [DiC₁₀][X] de formule 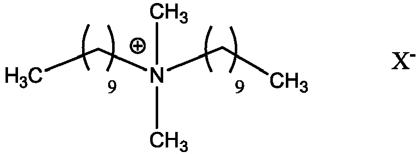 dans laquelle X⁻ a les significations désignées ci-dessus,
• des sels de trialkylammonium, en particulier des halogénures de trialkylammonium, notamment le bromure d'hexadécyl triméthylammonium (CTaBr) de formule H₃C(CH₂)₁₅N⁺(CH₃)₃,
• des sels de pyridinium, notamment le sel de cétylpyridinium de formule 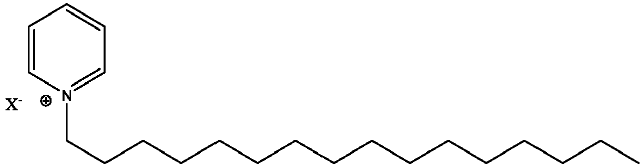 dans laquelle
X⁻ a les significations désignées ci-dessus,
• des sels de benzalkonium notamment des sels de formule 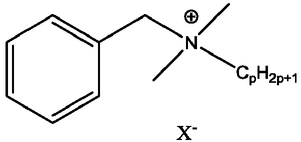 dans laquelle
X⁻ a les significations désignées ci-dessus,
p est compris de 8 à 18,
• des sels d'ammonium de la triéthanolamine de formule
(HO-CH₂-CH₂)₃NH⁺ X⁻,
dans laquelle
X⁻ a les significations désignées ci-dessus,
ou dans lesquelles les tensioactifs sont amphotères et sont choisis parmi
• les bétaïnes et représentent notamment la bétaïne de cocamidopropyle (CAPB) de formule, 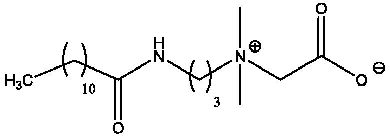
• ou les oxydes d'amine et représentent notamment l'oxyde de lauryldiméthylamine de formule 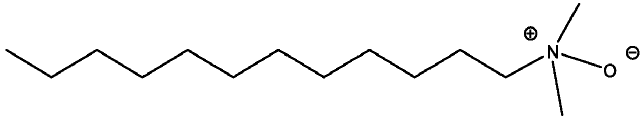 ou dans lesquelles les tensioactifs sont non-ioniques et sont choisis parmi
• les alcanolamides, notamment le monoéthanolamide (cocamide, MEA) de formule 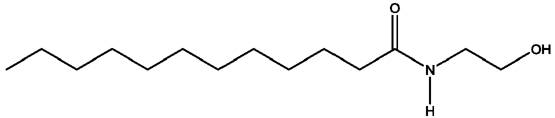 sous réserve que ledit alcanolamide ne soit pas un polyol,
• les alkylpolyglycosides (APG),
• les éthers de polyglycérol,
• ou les esters de polyglycérol.

7. Microémulsions selon l'une des revendications 4 à 6, dans lesquelles le rapport massique entre la substance parfumante ou la composition parfumante et le ou les solvo-surfactif(s) varie de 0,5 à 1,
et notamment dans lesquelles le rapport massique entre la substance parfumante définie ci-dessus ou le ou les tensioactif(s) varie de 5 à 100, notamment de 2,5 à 20.

8. Microémulsions, selon la revendication 1,
constituées de :
• 87,67% d'eau, 4,75% de composition parfumante P, 6,64% de C₆E₄, 0,94% d'un propoxysulfate de formule : 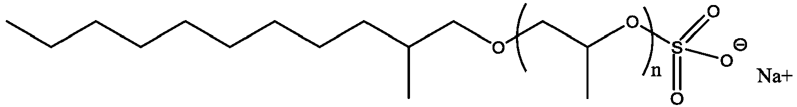 dans laquelle le nombre de motifs propoxylate n est 8,
• 86,04% d'eau, 5,00% de composition parfumante P, 8,01% de C₆E₄, 0,95% d'un propoxysulfate de formule : 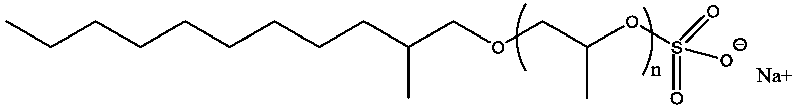 dans laquelle le nombre de motifs propoxylate n est 4,
• 87,69% d'eau, 4,77% de composition parfumante P, 6,45% de C₆E₄, 1,09% de dihéxylsulfosuccinate,
• 87,41% d'eau, 4,82% de composition parfumante P, 6,65% de C₆E₄, 1,12% de chlorure de didécyldiméthyl ammonium,
• 88,86% d'eau, 4,81% de composition parfumante PF1, 5,39% de C₆E₄, 0,94% d'un propoxysulfate de formule : 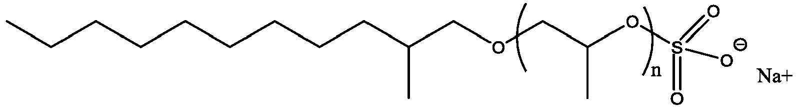 dans laquelle le nombre de motifs propoxylate n est 8,
• 86,08% d'eau, 4,89% de composition parfumante PF1, 8,00% de C₆E₄, 1,03% d'un propoxysulfate de formule : 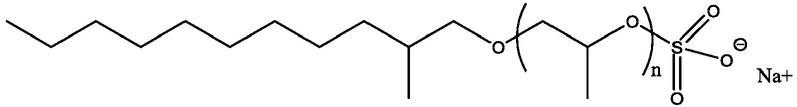 dans laquelle le nombre de motifs propoxylate n est 4,
• 87,75% d'eau, 4,87% de composition parfumante PF1, 6,16% de C₆E₄, 1,22% de dihéxylsulfosuccinate,
• 88,87% d'eau, 4,90% de composition parfumante PF1, 5,18% de C₆E₄, 1,05% de chlorure de didécyldiméthyl ammonium,
• 82,49% d'eau, 4,49% de composition parfumante PF1, 12,1% de C₆E₄, 0,92% de bétaïne de cocamidopropyle,
• 87,36% d'eau, 4,79% de composition parfumante PF2, 6,95% de C₆E₄, 0,90% d'un propoxysulfate de formule : 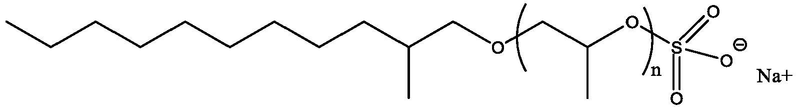 dans laquelle le nombre de motifs propoxylate n est 4,
• 86,66% d'eau, 4,90% de composition parfumante PF2, 7,30% de C₆E₄, 1,14% de dihéxylsulfosuccinate,
• 86,54% d'eau, 5,25% de composition parfumante PF2, 7,11% de C₆E₄, 1,10% de chlorure de didécyldiméthyl ammonium,
• 87,71% d'eau, 4,84% de composition parfumante PF3, 6,29% de C₆E₄, 1,16% de chlorure de didécyldiméthyl ammonium,
• 84,81% d'eau, 4,88% de composition parfumante PF3, 9,21% de C₆E₄, 1,10% de dihéxylsulfosuccinate,
• 86,25% d'eau, 4,88% de composition parfumante PF3, 7,90% de C₆E₄, 0,97% d'un propoxysulfate de formule : 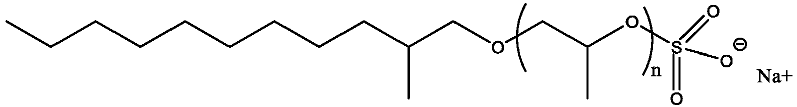 dans laquelle le nombre de motifs propoxylate n est 4,
• 86,91% d'eau, 4,85% de composition parfumante PF3, 7,28% de C₆E₄, 0,96% d'un propoxysulfate de formule : 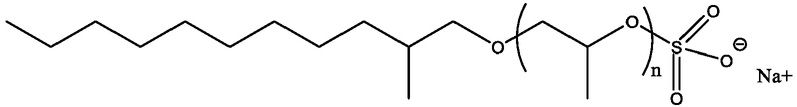 dans laquelle le nombre de motifs propoxylate n est 8,
dans lesquelles
• P est une composition parfumante complexe,
• PF1 est une composition parfumante contenant, en pourcentages massiques, 1,4% d'a-pinène, 1,5% de β-pinène, 0,4% de camphène, 21,3% de cinéole, 2,7% d'ocimène, 7,7% de camphre, 19,2% de dipropylène glycol, 36,7% de linalol, 7,4% d'acétate de linalyle et 1,7% de limonène,
• PF2 est une composition parfumante contenant, en pourcentages massiques, 1,4% d'a-pinène, 1,5% de β-pinène, 0,4% de camphène, 21,3% de cinéole, 2,7% d'ocimène, 7,7% de camphre, 19,2% de diéthylphtalate, 36,7% de linalol, 7,4% d'acétate de linalyle et 1,7% de limonène,
• PF3 est une composition parfumante contenant, en pourcentages massiques, 1,4% d'a-pinène, 1,5% de β-pinène, 0,4% de camphène, 21,3% de cinéole, 2,7% d'ocimène, 7,7% de camphre, 19,2% de myristate d'isopropyle, 36,7% de linalol, 7,4% d'acétate de linalyle et 1,7% de limonène.

9. Microémulsions, selon la revendication 1,
constituées de :
• 80,50% d'eau, 10,38% de composition parfumante PF1, 7,48% de C₆E₄, 1,64% de chlorure de didécyldiméthyl ammonium,
• 85,70% d'eau, 3,62% de composition parfumante PF1, 9,74% de C₆E₂, 0,95% d'un propoxysulfate de formule : 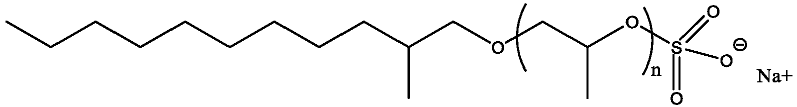 dans laquelle le nombre de motifs propoxylate n est 4,
• 85,43% d'eau, 3,59% de composition parfumante PF1, 9,56% de C₆E₂, 1,42% d'un propoxysulfate de formule : 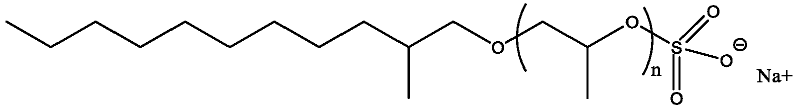 dans laquelle le nombre de motifs propoxylate n est 8,
• 85,29% d'eau, 4,74% de composition parfumante PF1, 9,50% de C₆E₃, 0,47% d'un propoxysulfate de formule : 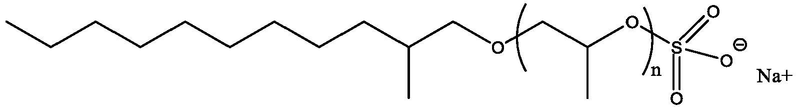 dans laquelle le nombre de motifs propoxylate n est 4,
• 85,33% d'eau, 4,73% de composition parfumante PF1, 9,44% de C₆E₃, 0,51% d'un propoxysulfate de formule : 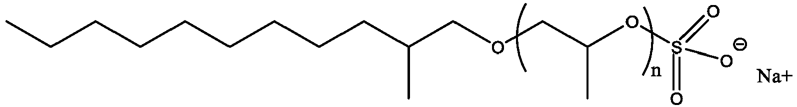 dans laquelle le nombre de motifs propoxylate n est 8,
• 85,32% d'eau, 4,71% de composition parfumante PF1, 9,80% de C₆E₃, 0,17% de dihéxylsulfosuccinate,
• 85,42% d'eau, 4,73% de composition parfumante PF1, 9,37% de C₆E₃, 0,48% d'un propoxysulfate de formule : 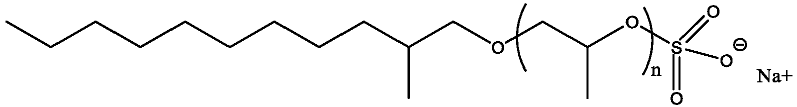 dans laquelle le nombre de motifs propoxylate n est 8,
• 85,25% d'eau, 4,74% de composition parfumante PF1, 9,54% de C₆E₃, 0,46% de bromure d'hexadécyl triméthylammonium,
• 85,03% d'eau, 4,74% de composition parfumante PF1, 9,75% de C₆E₃, 0,49% de dodécylbenzène sulfonate de sodium,
• 85,12% d'eau, 4,71% de composition parfumante PF1, 9,69% de C₆E₃, 0,48% de chlorure de didécyldiméthyl ammonium,
dans lesquelles
• PF1 est une composition parfumante contenant, en pourcentages massiques, 1,4% d'a-pinène, 1,5% de β-pinène, 0,4% de camphène, 21,3% de cinéole, 2,7% d'ocimène, 7,7% de camphre, 19,2% de dipropylène glycol, 36,7% de linalol, 7,4% d'acétate de linalyle et 1,7% de limonène.

10. Utilisation de microémulsions selon l'une des revendications 1 à 9, pour la préparation de compositions appliquées à :
• la parfumerie fine,
• la détergence, le textile et le nettoyage des surfaces dures,
• la cosmétique et les produits d'hygiène corporelle,
• la désinfection,
• le phytosanitaire,
• la pharmacie.

## Patentansprüche

1. Mikroemulsionen vom Typ Öl/Wasser, bestehend, in Masseprozent, aus:
- mindestens 80 % Wasser,
- 1 % bis 10 % hydrophoben Substanzen, insbesondere 3 % bis 10 %, insbesondere ungefähr 10 %,
- 5 % bis 15 %, insbesondere 5 % bis 10 %, eines oder mehrerer Solvotenside, deren Siedetemperatur weniger als 250°C bei Atmosphärendruck beträgt,
- 0,1 % bis 2 % eines oder mehrerer Tenside, in Masseprozent, wobei die Zusammensetzung, enthaltend die hydrophobe Substanz, Wasser, das Solvotensid und das Tensid, höchstens 2 %, bezogen auf die Gesamtmasse der Zusammensetzung, Verbindungen enthält, deren Siedetemperatur höher ist als 250°C bei Atmosphärendruck,
wobei eines der Solvotenside eine amphiphile Verbindung ist, ausgewählt aus:
• polyethoxylierten Fettalkoholen CᵢEⱼ mit einer linearen Kohlenstoffkette mit der Formel CH₃-(CH₂)ᵢ₋₁-(O-CH₂-CH₂)ⱼ-OH,
oder polyethoxylierten Fettalkoholen mit einer verzweigten Kette mit der Formel CᵢH₂ᵢ₊₁(OCH₂CH₂)ⱼOH,
wobei
- i die Anzahl der Kohlenstoffatome der linearen oder verzweigten Kohlenwasserstoffkette darstellt und von 4 bis 6 variiert,
- j die Anzahl von Ehtylenoxiden darstellt und von 2 bis 4 variiert,
wobei das Tensid ausgewählt ist aus
- anionischen Tensiden,
- kationischen Tensiden,
- amphoteren Tensiden, und
- nicht-ionischen Tensiden.

2. Mikroemulsionen vom Typ Öl/Wasser, bestehend, in Masseprozent, aus:
- mindestens 80 % Wasser,
- 1 % bis 10 % hydrophoben Substanzen, insbesondere 3 % bis 10 %, insbesondere ungefähr 10 %,
- 5 % bis 15 %, insbesondere 5 % bis 10 %, eines oder mehrerer Solvotenside, deren Siedetemperatur weniger als 250°C bei Atmosphärendruck beträgt,
- 0,1 % bis 2 % eines oder mehrerer Tenside, in Masseprozent, wobei die Zusammensetzung, enthaltend die hydrophobe Substanz, Wasser, das Solvotensid und das Tensid, höchstens 2 %, bezogen auf die Gesamtmasse der Zusammensetzung, Verbindungen enthält, deren Siedetemperatur höher ist als 250°C bei Atmosphärendruck,
wobei eines der Solvotenside eine amphiphile Verbindung ist, ausgewählt aus:
C₄E₁, C₅E₁, C₅E₂, C₅E₃, C₆E₂, C₆E₃, C₆E₄,
wobei das Tensid ausgewählt ist aus
- anionischen Tensiden,
- kationischen Tensiden,
- amphoteren Tensiden, und
- nicht-ionischen Tensiden.

3. Mikroemulsionen nach einem der Ansprüche 1 oder 2, wobei die hydrophobe Substanz eine parfümierende Substanz ist, ausgewählt aus einem Terpen, einem Terpenoid, einem ätherischen Öl oder einer synthetischen Verbindung, die Duftstoffeigenschaften aufweist, insbesondere ausgewählt aus Aldehyden, Estern, Ketonen, Iononen, Ethern,
wobei die parfümierende Substanz eine komplexe parfümierende Zusammensetzung sein kann, die eine Mischung mehrerer Terpene, Terpenoide, ätherischer Öle oder mehrerer reiner synthetischer Duftstoffverbindungen enthält,
wobei der Masseprozentsatz der parfümierenden Substanz zwischen 1 % und 10 %, und insbesondere 3 % und 10 %, liegt und insbesondere ungefähr gleich 5 % oder ungefähr gleich 10 % ist.

4. Mikroemulsionen nach einem der Ansprüche 1 bis 3, wobei die Solvotenside amphiphile Verbindungen sind, ausgewählt aus C₅E₂, C₆E₂, C₆E₃ oder C₆E₄.

5. Mikroemulsionen nach einem der Ansprüche 1 bis 4, wobei die Solvotenside in einer Mischung von mindestens zwei Solvotensid-Verbindungen verwendet werden,
wobei die Mischung insbesondere besteht aus mindestens 2 zwei CᵢEⱼ,
oder aus einem CᵢEⱼ und mindestens einem Glycerin-Derivat CₖGly mit der Formel: 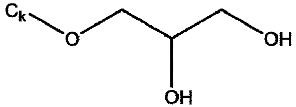 wobei Cₖ darstellt:
- eine lineare oder verzweigte Alkylgruppe mit 1 bis 6 Kohlenstoffatomen, insbesondere 3 bis 5 Kohlenstoffatomen, insbesondere 4 oder 5 Kohlenstoffatomen, insbesondere C₄Gly mit der Formel: 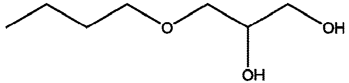 oder C₅Gly mit der Formel: 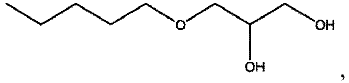 oder insbesondere das verzweigte Derivat i-C₅Gly mit der Formel: 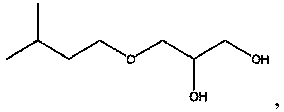
- eine lineare oder verzweigte Acylgruppe mit 2 bis 6 Kohlenstoffatomen, insbesondere 3 bis 5 Kohlenstoffatomen, insbesondere 4 oder 5 Kohlenstoffatomen, insbesondere Glycerylpentanoat mit der Formel: 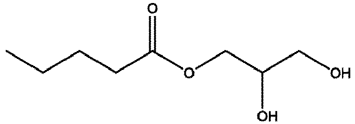 oder aus einem CᵢEⱼ und mindestens einem Isosorbid-, Isomannid- oder Isoidid-Derivat,
wobei die Mischung insbesondere aus C₆E₂ und C₄Gly in Volumenmengen von 1:1 besteht.

6. Mikroemulsionen nach einem der Ansprüche 1 bis 5, wobei die Tenside anionisch sind und darstellen:
- Alkylsulfonate, insbesondere Dihexylsulfosuccinat (DHS) mit der Formel: 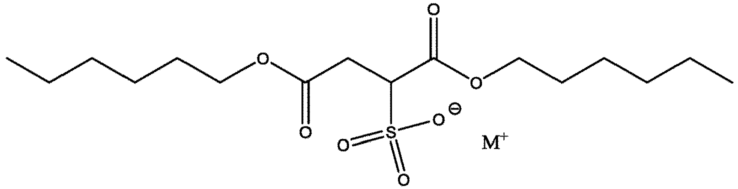 wobei
M⁺ Na⁺, K⁺, NH₄⁺, (HOCH₂CH₂)₃NH⁺ darstellt,
oder insbesondere Ethyl-2-hexylsulfonsuccinat mit der Formel: 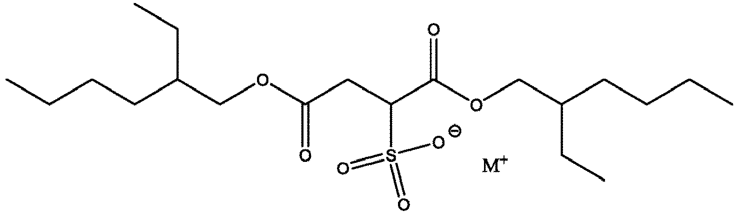 wobei M⁺ Na⁺, K⁺, NH₄⁺, (HOCH₂CH₂)₃NH⁺ darstellt,
- Alkylarylsulfonate mit der Formel: 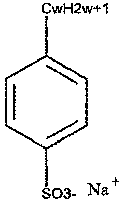 wobei w eine ganze Zahl von 8 bis 12 ist, insbesondere Natriumisooctyl-, -isononyl- und insbesondere -isodecyclbenzolsulfonat SDBS mit der Formel: 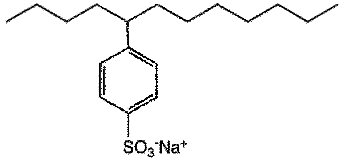
- Propoxysulfate, insbesondere Verbindungen mit der Formel: 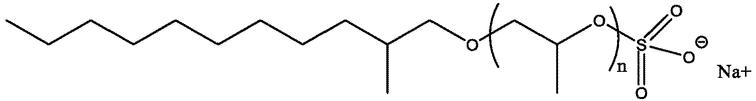 wobei die Anzahl der Propoxylat-Einheiten n von 4 bis 8 beträgt,
• wobei n insbesondere gleich 4 sein kann (Alf4S),
• wobei n insbesondere gleich 8 sein kann (Alf8S),
- oder Alkylsulfate, insbesondere Laurylsulfatsalze, wie Natriumdodecylsulfat (SDS), und Natriumalkylethersulfate, wie Natriumlaurylethersulfat (LESNa),
- oder Fettsäuresalze mit der Formel R-CO₂⁻M⁺, wobei R eine lineare oder verzweigte, gesättigte oder ungesättigte Kohlenstoffkette mit 8 bis 18 Kohlenstoffatomen darstellt, und M⁺ ein Kation darstellt, ausgewählt aus den Ionen Na⁺, K⁺, NH₄⁺, (HOCH₂CH₂)₃NH⁺,
insbesondere das Oleinsäuresalz mit der Formel CH₃(CH₂)₇CH=CH(CH₂)₇CO₂⁻M⁺, wobei M⁺ die oben definierten Bedeutungen hat,
oder wobei die Tenside kationisch sind und Ammoniumsalze sind, ausgewählt aus:
- Tetraalkylammoniumsalzen, insbesondere Dialkyldimethylammoniumsalzen mit der Formel: 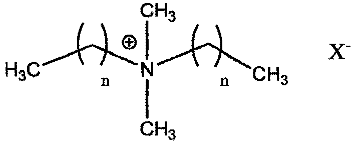 wobei
X⁻ ein Anion darstellt, ausgewählt aus Ci⁻, Br⁻, I⁻, CH₃COO⁻ oder Lactat,
n die Methylenzahl von 6 bis 12 ist,
und insbesondere ein Didecyldimethylammoniumsalz [DiC₁₀] [X] mit der Formel darstellt: 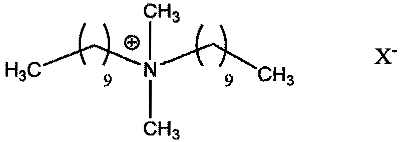 wobei X⁻ die oben angegebenen Bedeutungen hat,
- Trialkylammoniumsalzen, insbesondere Trialkylammoniumhalogeniden, insbesondere Hexadecyltrimethylammoniumbromid (CTaBr) mit der Formel H₃C(CH₂)₁₅NH⁺(CH₃)₃,
- Pyridiniumsalzen, insbesondere das Cetylpyridiniumsalz mit der Formel: 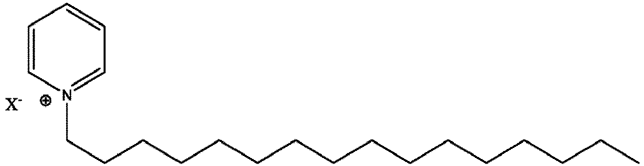 wobei
X⁻ die oben angegebenen Bedeutungen hat,
- Benzalkoniumsalzen, insbesondere den Salzen mit der Formel: 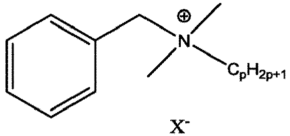 wobei
X⁻ die oben angegebenen Bedeutungen hat,
p von 8 bis 18 beträgt,
- Ammoniumsalzen von Triethanolamin mit der Formel:
(HO-CH₂-CH₂)₃NH⁺ X⁻,
wobei
X⁻ die oben angegebenen Bedeutungen hat,
oder wobei die Tenside amphoter sind und ausgewählt sind aus:
- Betainen und insbesondere Cocamidopropylbetain (CAPB) mit der Formel darstellen: 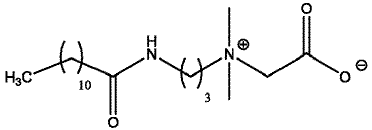
- oder Aminoxiden und insbesondere Lauryldimethylaminoxid mit der Formel darstellen: 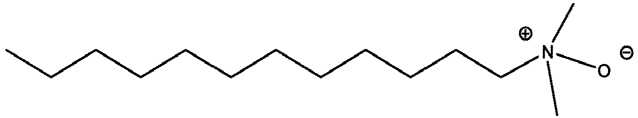 oder wobei die Tenside nicht-ionisch sind und ausgewählt sind aus:
- Alkanolamiden, insbesondere Monoethanolamid (Cocamid, MEA) mit der Formel: 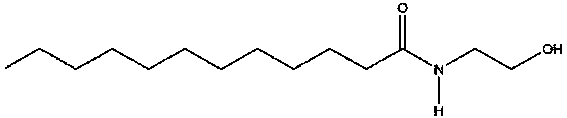 mit der Maßgabe, dass das Alkanolamid kein Polyol ist,
- Alkylpolyglykosiden (APG),
- Polyglycerinethern,
- oder Polyglycerinestern.

7. Mikroemulsionen nach einem der Ansprüche 4 bis 6, wobei das Masseverhältnis zwischen der parfümierenden Substanz oder der parfümierenden Zusammensetzung und dem oder den Solvotensid(en) von 0,5 bis 1 variiert, und wobei insbesondere das Masseverhältnis zwischen der oben definierten parfümierenden Substanz und dem oder den Tensid(en) von 5 bis 100, insbesondere von 2,5 bis 20 variiert.

8. Mikroemulsionen nach Anspruch 1,
bestehend aus:
- 87,67 % Wasser, 4,75 % der parfümierenden Zusammensetzung P, 6,64 % C₆E₄, 0,94 % eines Propoxysulfats mit der Formel: 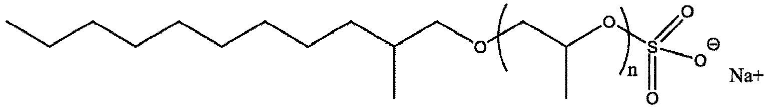 wobei die Anzahl der Propoxylat-Einheiten n 8 beträgt,
- 86,04 % Wasser, 5,00 % der parfümierenden Zusammensetzung P, 8,01 % C₆E₄, 0,95 % eines Propoxysulfats mit der Formel: 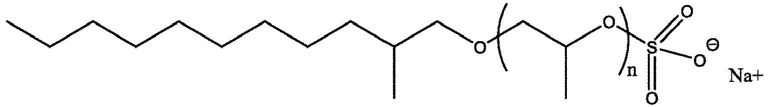 wobei die Anzahl der Propoxylat-Einheiten n 4 beträgt,
- 87,69 % Wasser, 4,77 % der parfümierenden Zusammensetzung P, 6,45 % C₆E₄, 1,09 % Dihexylsulfosuccinat,
- 87,41 % Wasser, 4,82 % der parfümierenden Zusammensetzung P, 6,65 % C₆E₄, 1,12 % Didecyldimethylammoniumchlorid,
- 88,86 % Wasser, 4,81 % der parfümierenden Zusammensetzung PF1, 5,39 % C₆E₄, 0,94 % eines Propoxysulfats mit der Formel: 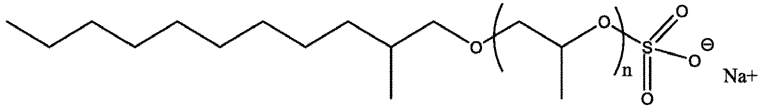 wobei die Anzahl der Propoxylat-Einheiten n 8 beträgt,
- 86,08 % Wasser, 4,89 % der parfümierenden Zusammensetzung PF1, 8,00 % C₆E₄, 1,03 % eines Propoxysulfats mit der Formel: 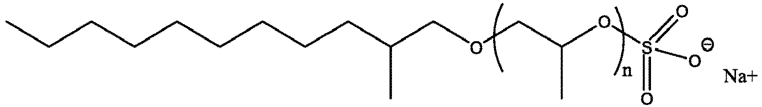 wobei die Anzahl der Propoxylat-Einheiten n 4 beträgt,
- 87,75 % Wasser, 4,87 % der parfümierenden Zusammensetzung PF1, 6,16 % C₆E₄, 1,22 % Dihexylsulfosuccinat,
- 88,87 % Wasser, 4,90 % der parfümierenden Zusammensetzung PF1, 5,18 % C₆E₄, 1,05 % Didecyldimethylammoniumchlorid,
- 82,49 % Wasser, 4,49 % der parfümierenden Zusammensetzung PF1, 12,1 % C₆E₄, 0,92 % Cocamidopropylbetain,
- 87,36 % Wasser, 4,79 % der parfümierenden Zusammensetzung PF2, 6,95 % C₆E₄, 0,90 % eines Propoxysulfats mit der Formel: 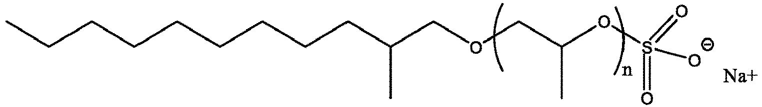 wobei die Anzahl der Propoxylat-Einheiten n 4 beträgt,
- 86,66 % Wasser, 4,90 % der parfümierenden Zusammensetzung PF2, 7,30 % C₆E₄, 1,14 % Dihexylsulfosuccinat,
- 86,54 % Wasser, 5,25 % der parfümierenden Zusammensetzung PF2, 7,11 % C₆E₄, 1,10 % Didecyldimethylammoniumchlorid,
- 87,71 % Wasser, 4,84 % der parfümierenden Zusammensetzung PF3, 6,29 % C₆E₄, 1,16 % Didecyldimethylammoniumchlorid,
- 84,81 % Wasser, 4,88 % der parfümierenden Zusammensetzung PF3, 9,21 % C₆E₄, 1,10 % Dihexylsulfosuccinat,
- 86,25 % Wasser, 4,88 % der parfümierenden Zusammensetzung PF3, 7,90 % C₆E₄, 0,97 % eines Propoxysulfats mit der Formel: 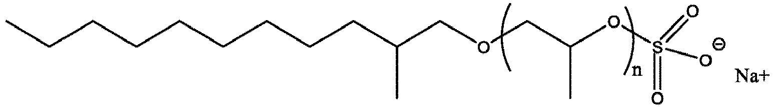 wobei die Anzahl der Propoxylat-Einheiten n 4 beträgt,
- 86,91 % Wasser, 4,85 % der parfümierenden Zusammensetzung PF3, 7,28 % C₆E₄, 0,96 % eines Propoxysulfats mit der Formel: 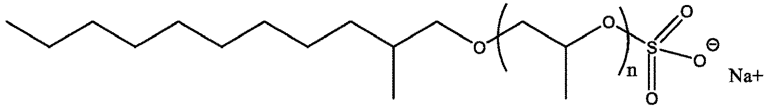 wobei die Anzahl der Propoxylat-Einheiten n 8 beträgt, wobei
- P eine komplexe parfümierende Zusammensetzung ist,
- PF1 eine parfümierende Zusammensetzung ist, die, in Masseprozent, 1,4 % α-Pinen, 1,5 % β-Pinen, 0,4 % Camphen, 21,3 % Cineol, 2,7 % Ocimen, 7,7 % Campher, 19,2 % Dipropylenglykol, 36,7 % Linalol, 7,4 % Linalylacetat und 1,7 % Limonen enthält,
- PF2 eine parfümierende Zusammensetzung ist, die, in Masseprozent, 1,4 % α-Pinen, 1,5 % β-Pinen, 0,4 % Camphen, 21,3 % Cineol, 2,7 % Ocimen, 7,7 % Campher, 19,2 % Diethylphthalat, 36,7 % Linalol, 7,4 % Linalylacetat und 1,7 % Limonen enthält,
- PF3 eine parfümierende Zusammensetzung ist, die, in Masseprozent, 1,4 % α-Pinen, 1,5 % β-Pinen, 0,4 % Camphen, 21,3 % Cineol, 2,7 % Ocimen, 7,7 % Campher, 19,2 % Isopropylmyristat, 36,7 % Linalol, 7,4 % Linalylacetat und 1,7 % Limonen enthält.

9. Mikroemulsionen nach Anspruch 1,
bestehend aus:
- 80,50 % Wasser, 10,38 % der parfümierenden Zusammensetzung PF1, 7,48 % C₆E₄, 1,64 % Didecyldimethylammoniumchlorid,
- 85,70 % Wasser, 3,62 % der parfümierenden Zusammensetzung PF1, 9,74 % C₆E₂, 0,95 % eines Propoxysulfats mit der Formel: 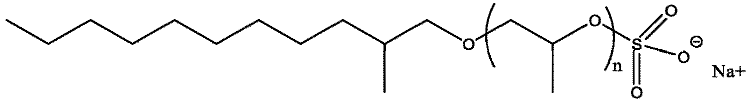 wobei die Anzahl der Propoxylat-Einheiten n 4 beträgt,
- 85,43 % Wasser, 3,59 % der parfümierenden Zusammensetzung PF1, 9,56 % C₆E₂, 1,42 % eines Propoxysulfats mit der Formel: 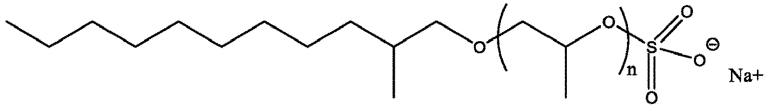 wobei die Anzahl der Propoxylat-Einheiten n 8 beträgt,
- 85,29 % Wasser, 4,74 % der parfümierenden Zusammensetzung PF1, 9,50 % C₆E₃, 0,47 % eines Propoxysulfats mit der Formel: 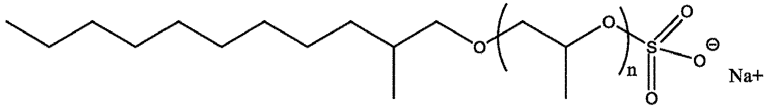 wobei die Anzahl der Propoxylat-Einheiten n 4 beträgt,
- 85,33 % Wasser, 4,73 % der parfümierenden Zusammensetzung PF1, 9,44 % C₆E₃, 0,51 % eines Propoxysulfats mit der Formel: 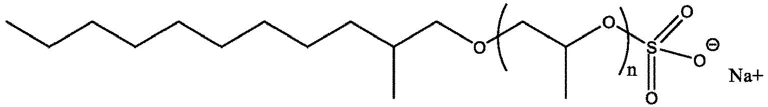 wobei die Anzahl der Propoxylat-Einheiten n 8 beträgt,
- 85,32 % Wasser, 4,71 % der parfümierenden Zusammensetzung PF1, 9,80 % C₆E₃, 0,17 % Dihexylsulfonsuccinat,
- 85,42 % Wasser, 4,73 % der parfümierenden Zusammensetzung PF1, 9,37 % C₆E₃, 0,48 % eines Propoxysulfats mit der Formel: 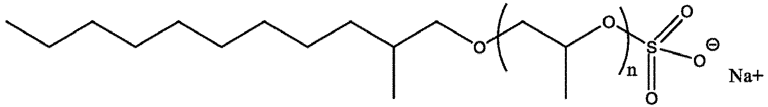 wobei die Anzahl der Propoxylat-Einheiten n 8 beträgt,
- 85,25 % Wasser, 4,74 % der parfümierenden Zusammensetzung PF1, 9,54 % C₆E₃, 0,46 % Hexadecyltrimethylammoniumbromid,
- 85,03 % Wasser, 4,74 % der parfümierenden Zusammensetzung PF1, 9,75 % C₆E₃, 0,49 % Natriumdodecylbenzolsulfonat,
- 85,12 % Wasser, 4,71 % der parfümierenden Zusammensetzung PF1, 9,69 % C₆E₃, 0,48 % Didecyldimethylammoniumchlorid,
wobei
- PF1 eine parfümierende Zusammensetzung ist, die, in Masseprozent, 1,4 % α-Pinen, 1,5 % β-Pinen, 0,4 % Camphen, 21,3 % Cineol, 2,7 % Ocimen, 7,7 % Campher, 19,2 % Dipropylenglykol, 36,7 % Linalol, 7,4 % Linalylacetat und 1,7 % Limonen enthält.

10. Verwendung der Mikroemulsionen nach einem der Ansprüche 1 bis 9 zur Herstellung von Zusammensetzungen, die angewendet werden:
- in der feinen Parfümerie,
- in Waschmitteln, Textilien und bei der Reinigung harter Oberflächen,
- in der Kosmetik und Körperhygieneprodukten,
- in der Desinfektion,
- im Pflanzenschutz,
- in der Pharmazie.

## Claims

1. Microemulsions of oil/water type consisting in, in weight percentage:
• at least 80% of water,
• 1% to 10% of hydrophobic substances, in particular 3% to 10%, notably about 10%,
• 5% to 15%, notably 5% to 10%, of one or more solvo-surfactants having a boiling point lower than 250°C at atmospheric pressure,
• 0,1% to 2% of one or more surfactants, in weight percentage,
the composition containing the hydrophobic substance, water, the solvo-surfactant and the surfactant containing at most 2% of the total weight of the composition of compounds having a boiling point higher than 250°C at atmospheric pressure,
one of the solvo-surfactant being an amphiphilic compound chosen from :
▪ polyethoxylated fatty alcohols CᵢEⱼ with a linear carbon chain, of formula CH₃-(CH₂)ᵢ₋₁-(O-CH₂-CH₂)ⱼ-OH,
or polyethoxylated fatty alcohols with a branched chain, of formula CᵢH₂ᵢ₊₁(OCH₂CH₂)ⱼOH, in which
• i represents the number of carbon atoms in the linear or branched carbon chain and varies from 4 to 6,
• j represents the number of ethylene oxides and varies from 2 to 4,
said surfactant is chosen among
• anionic surfactants,
• cationic surfactants,
• amphoteric surfactants, and
• non-ionic surfactants.

2. Microemulsions of oil/water type consisting in, in weight percentage:
• at least 80% of water,
• 1% to 10% of hydrophobic substances, in particular 3% to 10%, notably about 10%,
• 5% to 15%, notably 5% to 10%, of one or more solvo-surfactants having a boiling point lower than 250°C at atmospheric pressure,
• 0,1% to 2% of one or more surfactants, in weight percentage,
the composition containing the hydrophobic substance, water, the solvo-surfactant and the surfactant containing at most 2% of the total weight of the composition of compounds having a boiling point higher than 250°C at atmospheric pressure,
one of the solvo-surfactant being an amphiphilic compound chosen from :
C₄E₁, C₅E₁, C₅E₂, C₅E₃, C₆E₂, C₆E₃, C₆E₄,
said surfactant is chosen among
• anionic surfactants,
• cationic surfactants,
• amphoteric surfactants, and
• non-ionic surfactants.

3. Microemulsions according to anyone of claims 1 or 2, in which the hydrophobic substance is a fragrant substance chosen among a terpene, a terpenoid, an essential oil, or a synthetic compound having fragrant properties, notably chosen among aldehydes, esters, ketones, ionones, ethers,
said fragrant substance can be a complex fragrant composition, containing a mixture of several terpenes, terpenoids, essential oils, or several pure synthetic compounds having fragrant properties,
the weight percentage of said fragrant substance being from 1% to 10%, and notably from 3% to 10%, and in particular approximately equal to 5% or approximately equal to 10%.

4. Microemulsions according to anyone of claims 1 to 3, in which the solvo-surfactants are amphiphilic compounds chosen among C₅E₂, C₆E₂, C₆E₃ or C₆E₄.

5. Microemulsions according to anyone of claims 1 to 4, in which the solvo-surfactants are used as a mixture of at least two solvo-surfactant compounds,
the mixture being in particular constituted of at least two CᵢEⱼ,
or one CᵢEⱼ and at least one glycerol derivative CₖGly of formula : 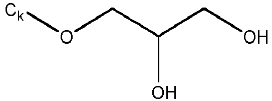 where Cₖ represents
• a linear or branched alkyl group, containing from 1 to 6 carbon atoms, in particular from 3 to 5 carbon atoms, notably 4 or 5 carbon atoms,
notably C₄Gly of formula 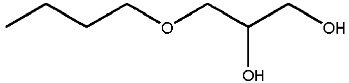 or C₅ Gly of formula 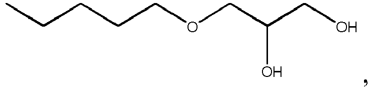 or notably the branched derivative i-C₅Gly of formula 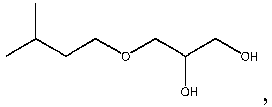
• a linear or branched acyl group, containing from 2 to 6 carbon atoms, in particular from 3 to 5 carbon atoms, notably 4 or 5 carbon atoms,
notably glyceryl pentanoate of formula 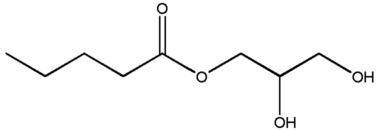 or a CᵢEⱼ and at least an isosorbide, isomannide, or isoidide derivative,
the mixture being notably constituted of C₆E₂ and C₄Gly in a ratio of 1 to 1 in volume.

6. Microemulsions according to anyone of claims 1 to 5, in which surfactants are anionic and represent
• alkylsulfonates, notably dihexyl sulfosuccinate (DHS) of formula 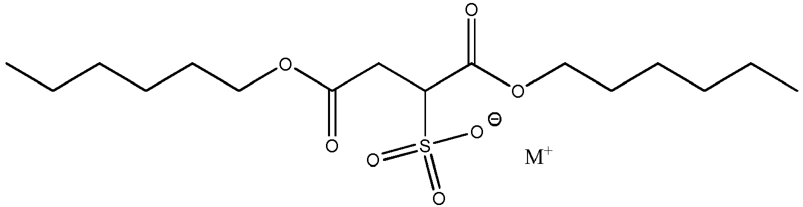 in which
M⁺ represents Na⁺, K⁺, NH₄⁺, (HOCH₂CH₂)₃NH⁺,
or notably ethyl-2-hexyl sulfosuccinate of formula 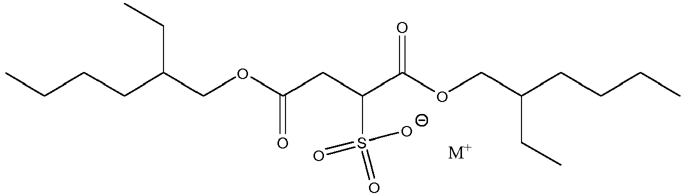 in which
M⁺ represents Na⁺, K⁺, NH₄⁺, (HOCH₂CH₂)₃NH⁺,
• alkylarylsulfonates of formula 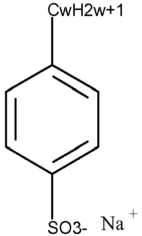 in which w in an integer from 8 to 12,
in particular isooctyl, isononyl and notably sodium isododecylbenzenesulfonate SDBS of formula 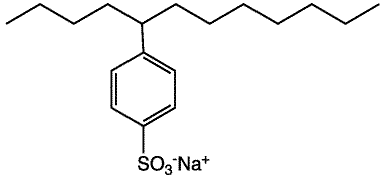
• propoxysulfates, notably compounds of formula 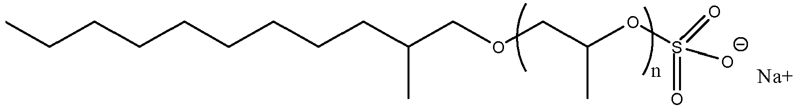 in which the number of propoxylate moieties n is from 4 to 8,
□ n can notably being 4, (Alf4S)
□ n can notably being 8, (Alf8S)
• or alkylsulfates, notably lauryl sulfate salts such as sodium dodecyl sulfate (SDS) and sodium alkylether sulfates such as sodium lauryl ether sulfate (LESNa),
• or fatty acid salts of formula R-CO₂⁻M⁺, in which R represents a linear or branched, saturated or insaturated carbon chain, containing 8 to 18 carbon atoms, and M⁺ represents a cation chosen among Na⁺, K⁺, NH₄⁺, (HOCH₂CH₂)₃NH⁺ ions,
notably the oleic acid salt of formula CH₃(CH₂)₇CH=CH(CH₂)₇CO₂⁻ M⁺ in which M⁺ has the above-mentioned definitions,
or in which surfactants are cationic and are ammonium salts chosen among
• tetraalkylammonium salts, notably dialkyldimethylammonium salts of formula 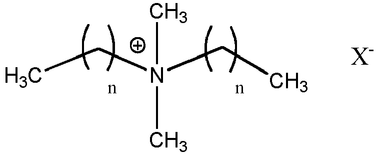 in which
X⁻ represents an anion chosen among Cl⁻, Br⁻, I⁻, CH₃COO⁻ or lactate,
n is the number of methylene from 6 to 12,
and represents notably a salt of didecyl dimethylammonium [DiC₁₀][X] of formula 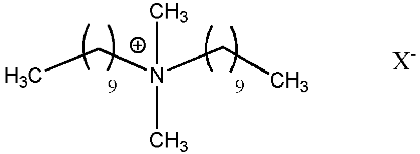 in which X⁻ has the above-mentioned definitions,
• trialkylammonium salts, in particular trialkylammonium halides, notably hexadecyl trimethylammonium bromide (CTaBr) of formula H₃C(CH₂)₁₅N⁺(CH₃)₃,
• pyridinium salts, notably the cetylpyridinium salt of formula 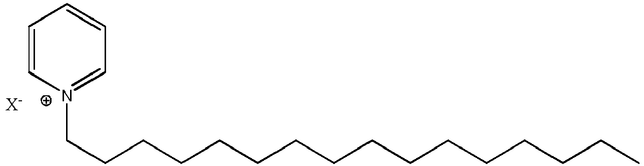 in which
X⁻ has the above-mentioned definitions,
• benzalkonium salts, notably salts of formula 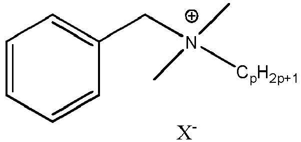 in which
X⁻ has the above-mentioned definitions,
p is from 8 to 18,
• triethanolamine ammonium salts of formula
(HO-CH₂-CH₂)₃NH⁺ X,
in which
X⁻ has the above-mentioned definitions,
or in which surfactants are amphoteric and are chosen among
• betaines and represent notably the cocamidopropyl betaine (CAPB) of formula, 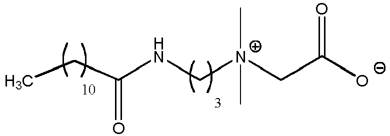
• or amine oxydes and represent notably lauryldimethylamine oxyde of formula, 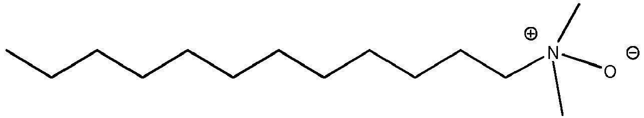 or in which surfactants are non-ionic and are chosen among
• alkanolamides, notably monoethanolamide (cocamide, MEA) of formula 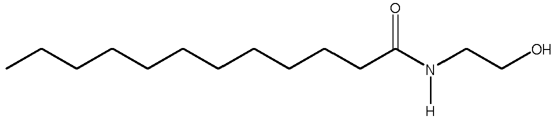 provided that said alkanolamide is not a polyol,
• alkylpolyglycosides (APG),
• polyglycerol ethers,
• or polyglycerol esters.

7. Microemulsions according to anyone of claims 4 to 6, in which the weight ratio between the fragrant substance or the fragrant composition and solvo-surfactant(s) varies from 0,5 to 1,
and notably in which the weight ratio between the above-defined fragrant substance and surfactant(s) varies from 5 to 100, notably from 2,5 to 20.

8. Microemulsions, according to claim 1,
constituted of :
• 87,67% of water, 4,75% of fragrant composition P, 6,64% of C₆E₄, 0,94% of a propoxysulfate of formula : 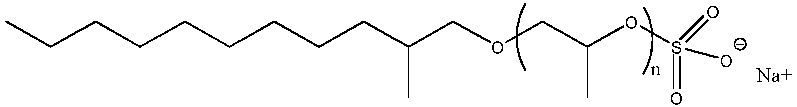 in which the number of propoxylate moieties n is 8,
• 86,04% of water, 5,00% of fragrant composition P, 8,01% of C₆E₄, 0,95% of a propoxysulfate of formula : 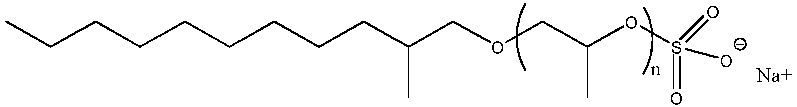 in which the number of propoxylate moieties n is 4,
• 87,69% of water, 4,77% of fragrant composition P, 6,45% of C₆E₄, 1,09% of dihexylsulfosuccinate,
• 87,41% of water, 4,82% of fragrant composition P, 6,65% of C₆E₄, 1,12% of didecyldimethyl ammonium chloride,
• 88,86% of water, 4,81% of fragrant composition PF1, 5,39% of C₆E₄, 0,94% of a propoxysulfate of formula : 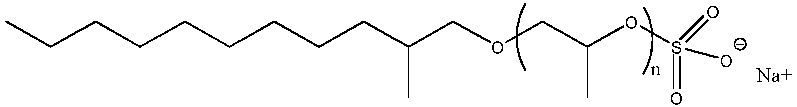 in which the number of propoxylate moieties n is 8,
• 86,08% of water, 4,89% of fragrant composition PF1, 8,00% of C₆E₄, 1,03% of a propoxysulfate of formula : 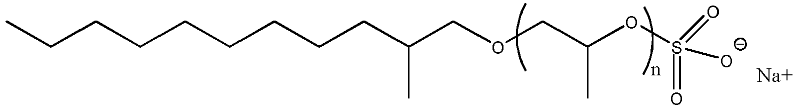 in which the number of propoxylate moieties n is 4,
• 87,75% of water, 4,87% of fragrant composition PF1, 6,16% of C₆E₄, 1,22% of dihexylsulfosuccinate,
• 88,87% of water, 4,90% of fragrant composition PF1, 5,18% of C₆E₄, 1,05% of didecyldimethyl ammonium chloride,
• 82,49% of water, 4,49% of fragrant composition PF1, 12,1% of C₆E₄, 0,92% of cocamidopropyl betaine,
• 87,36% of water, 4,79% of fragrant composition PF2, 6,95% of C₆E₄, 0,90% of a propoxysulfate of formula : 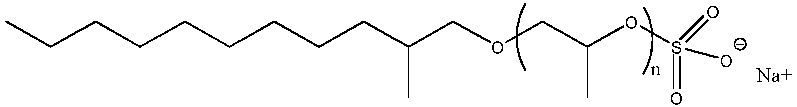 in which the number of propoxylate moieties n is 4,
• 86,66% of water, 4,90% of fragrant composition PF2, 7,30% of C₆E₄, 1,14% of dihexylsulfosuccinate,
• 86,54% of water, 5,25% of fragrant composition PF2, 7,11% of C₆E₄, 1,10% of didecyldimethyl ammonium chloride,
• 87,71% of water, 4,84% of fragrant composition PF3, 6,29% of C₆E₄, 1,16% of didecyldimethyl ammonium chloride,
• 84,81% of water, 4,88% of fragrant composition PF3, 9,21% of C₆E₄, 1,10% of dihexylsulfosuccinate,
• 86,25% of water, 4,88% of fragrant composition PF3, 7,90% de C₆E₄, 0,97% of a propoxysulfate of formula : 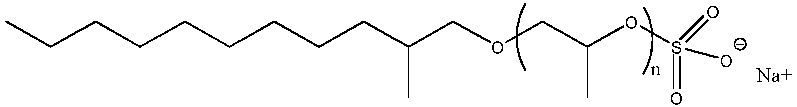 in which the number of propoxylate moieties n is 4,
• 86,91% of water, 4,85% of fragrant composition PF3, 7,28% of C₆E₄, 0,96% of a propoxysulfate of formula : 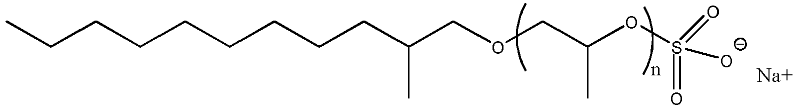 in which the number of propoxylate moieties n is 8,
in which
• P is a complex fragrant composition,
• PF1 is a fragrant composition containing, in weight percentages, 1,4% of α-pinene, 1,5% of β-pinene, 0,4% of camphene, 21,3% of cineole, 2,7% of ocimene, 7,7% of camphor, 19,2% of dipropylene glycol, 36,7% of linalool, 7,4% of linalyl acetate and 1,7% of limonene,
• PF2 is a fragrant composition containing, in weight percentages, 1,4% of α-pinene, 1,5% of β-pinene, 0,4% of camphene, 21,3% of cineole, 2,7% of ocimene, 7,7% of camphor, 19,2% of diethylphtalate, 36,7% of linalool, 7,4% of linalyl acetate and 1,7% of limonene,
• PF3 is a fragrant composition containing, in weight percentages, 1,4% of α-pinene, 1,5% of β-pinene, 0,4% of camphene, 21,3% of cineole, 2,7% of ocimene, 7,7% of camphor, 19,2% of isopropyl myristate, 36,7% of linalool, 7,4% of linalyl acetate and 1,7% of limonene.

9. Microemulsions, according to claim 1,
constituted of :
• 80,50% of water, 10,38% of fragrant composition PF1, 7,48% of C₆E₄, 1,64% of didecyldimethyl ammonium chloride,
• 85,70% of water, 3,62% of fragrant composition PF1, 9,74% of C₆E₂, 0,95% of a propoxysulfate of formula : 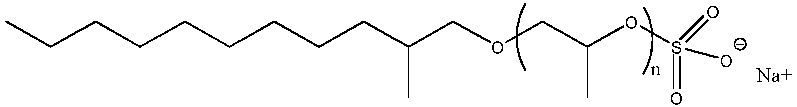 in which the number of propoxylate moieties n is 4,
• 85,43% of water, 3,59% of fragrant composition PF1, 9,56% of C₆E₂, 1,42% of a propoxysulfate of formula : 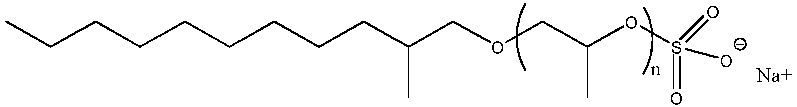 in which the number of propoxylate moieties n is 8,
• 85,29% of water, 4,74% of fragrant composition PF1, 9,50% of C₆E₃, 0,47% of a propoxysulfate of formula : 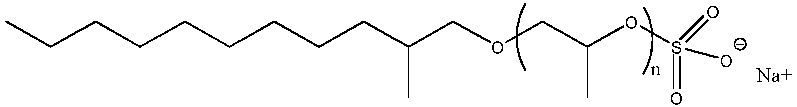 in which the number of propoxylate moieties n is 4,
• 85,33% of water, 4,73% of fragrant composition PF1, 9,44% of C₆E₃, 0,51% of a propoxysulfate of formula : 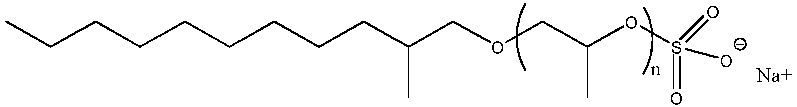 in which the number of propoxylate moieties n is 8,
• 85,32% of water, 4,71% of fragrant composition PF1, 9,80% of C₆E₃, 0,17% of dihexylsulfosuccinate,
• 85,42% of water, 4,73% of fragrant composition PF1, 9,37% of C₆E₃, 0,48% of a propoxysulfate of formula : 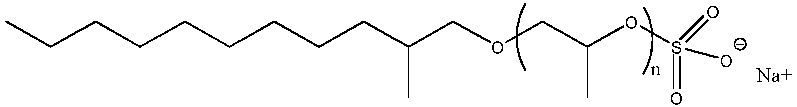 in which the number of propoxylate moieties n is 8,
• 85,25% of water, 4,74% of fragrant composition PF1, 9,54% of C₆E₃, 0,46% of hexadecyl trimethylammonium bromide,
• 85,03% of water, 4,74% of fragrant composition PF1, 9,75% of C₆E₃, 0,49% of sodium dodecylbenzene sulfonate,
• 85,12% of water, 4,71% of fragrant composition PF1, 9,69% of C₆E₃, 0,48% of didecyldimethyl ammonium chloride,
in which
• PF1 is a fragrant composition containing, in weight percentages, 1,4% of α-pinene, 1,5% of β-pinene, 0,4% of camphene, 21,3% of cineole, 2,7% of ocimene, 7,7% of camphor, 19,2% of dipropylene glycol, 36,7% of linalool, 7,4% of linalyl acetate and 1,7% of limonene.

10. Use of microemulsions according to anyone of claims 1 to 9, for the preparation of compositions applied to :
• fine fragrances,
• detergence, textile and cleaning of hard surfaces,
• cosmetics and personal care products,
• disinfection,
• agrochemicals,
• drugs.
